# EUROPEAN PATENT SPECIFICATION

(11) **EP 3 148 575 B1**
(45) Date of publication and mention of the grant of the patent: **21.08.2019**
(21) Application number: 15803012.2
(22) Date of filing: 02.06.2015
(51) Int. Cl.: A61K 47/68, A61K 47/54, A61P 37/08, C07K 16/28, C07K 5/10

(54) **COMPOSITIONS FOR TREATING ALLERGY AND INFLAMMATORY DISEASES**
ZUSAMMENSETZUNGEN ZUR BEHANDLUNG VON ALLERGIE- UND ENTZÜNDUNGSKRANKHEITEN
COMPOSITIONS POUR LE TRAITEMENT DES MALADIES ALLERGIQUES ET INFLAMMATOIRES

(30) Priority: 02.06.2014 US 201462006575 P
(43) Date of publication of application: 05.04.2017
(73) Proprietor: Baylor Research Institute, Dallas, TX 75201 (US)
(72) Inventor: OH, Sangkon, Baltimore, MD 21237 (US); KANE, Bob, Dallas, TX 75204 (US); ZURAWSKI, Gerard, Midlothian, TX 76065 (US)
(74) Representative: J A Kemp
(86) International application number: PCT/US2015/033696
(87) International publication number: WO 2015/187637

(56) References cited:
- US-A1- 2012 231 023
- US-A1- 2012 231 023
- US-A1- 2013 052 193
- US-A1- 2013 052 193
- GERBEN FERWERDA ET AL: "Dectin-1 synergizes with TLR2 and TLR4 for cytokine production in human primary monocytes and macrophages", CELLULAR MICROBIOLOGY, vol. 10, no. 10, 1 October 2008 (2008-10-01), pages 2058-2066, XP055240799, GB ISSN: 1462-5814, DOI: 10.1111/j.1462-5822.2008.01188.x
- UPCHURCH KATHERINE C ET AL: "Preclinical Assessment of the Effectiveness of alpha-Dectin-1-Pam3 Conjugate in Controlling T(H)2 Responses", JOURNAL OF ALLERGY AND CLINICAL IMMUNOLOGY, vol. 135, no. 2, Suppl. S, February 2015 (2015-02), page AB101, XP002776641, & ANNUAL MEETING OF THE AMERICAN-ACADEMY-OF-ALLERGY-ASTHMA-AND-IMM UNOLOGY (AAAAI); HOUSTON, TX, USA; FEBRUARY 20 -24, 2015
- FERWERDA ET AL.: 'Dectin-1 synergizes with TLR2 and TLR4 for cytokine production in human primary monocytes and macrophages.' CELL MICROBIOL. vol. 10, no. 10, 2008, ISSN 1462-5814 pages 2058 - 2066, XP055240799
- DRAKE ET AL.: 'The therapeutic potential of Toll-like receptor 7 stimulation in asthma.' INFLAMM ALLERGY DRUG TARGETS. vol. 11, no. 6, 2012, ISSN 1871-5281 pages 484 - 491, XP055240800

### SEQUENCE LISTING

<110> Oh, Sangkon Kane, Bob Zurawski, Gerard
<120> METHODS AND COMPOSITIONS FOR TREATING ALLERGY AND INFLAMMATORY DISEASES
<130> BHCS.P0417WO
<140> UNKNOWN
   <141> 2015-06-02
<150> 62/006,575
   <151> 2014-06-02
<160> 30
<170> PatentIn version 3.5
<210> 1
   <211> 447
   <212> PRT
   <213> Homo sapiens
<400> 1
   Gln Val Gln Leu Lys Glu Ser Gly Pro Gly Leu Val Ala Pro Ser Gln
   1               5                   10                  15
   Ser Leu Ser Ile Thr Cys Ser Val Ser Gly Phe Ser Leu Ser Asn Tyr
               20                  25                  30
   Asp Ile Ser Trp Ile Arg Gln Pro Pro Gly Lys Gly Leu Glu Trp Leu
           35                  40                  45
   Gly Val Met Trp Thr Gly Gly Gly Ala Asn Tyr Asn Ser Ala Phe Met
       50                  55                  60
   Ser Arg Leu Ser Ile Asn Lys Asp Asn Ser Lys Ser Gln Val Phe Leu
   65                  70                  75                  80
   Lys Met Asn Asn Leu Gln Thr Asp Asp Thr Ala Ile Tyr Tyr Cys Val
                   85                  90                  95
   Arg Asp Ala Val Arg Tyr Trp Asn Phe Asp Val Trp Gly Ala Gly Thr
               100                 105                 110
   Thr Val Thr Val Ser Ser Ala Lys Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Lys Thr Tyr Thr Cys Asn Val Asp His Lys Pro Ser
           195                 200                 205
   Asn Thr Lys Val Asp Lys Arg Val Glu Ser Lys Tyr Gly Pro Pro Cys
       210                 215                 220
   Pro Pro Cys Pro Ala Pro Glu Phe Glu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser Gln Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Gly Leu Pro Ser Ser Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Gln Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys Ala Ser
   435 440 445
<210> 2
   <211> 122
   <212> **PRT**
   <213> Homo sapiens
<400> 2
   Gln Val Gln Leu Lys Glu Ser Gly Pro Gly Leu Val Ala Pro Ser Gln
   1 5 10 15
   Ser Leu Ser Ile Thr Cys Ser Val Ser Gly Phe Ser Leu Ser Asn Tyr
   20 25 30
   Asp Ile Ser Trp Ile Arg Gln Pro Pro Gly Lys Gly Leu Glu Trp Leu
   35 40 45
   Gly Val Met Trp Thr Gly Gly Gly Ala Asn Tyr Asn Ser Ala Phe Met
   50 55 60
   Ser Arg Leu Ser Ile Asn Lys Asp Asn Ser Lys Ser Gln Val Phe Leu
   65 70 75 80
   Lys Met Asn Asn Leu Gln Thr Asp Asp Thr Ala Ile Tyr Tyr Cys Val
   85 90 95
   Arg Asp Ala Val Arg Tyr Trp Asn Phe Asp Val Trp Gly Ala Gly Thr
   100 105 110
   Thr Val Thr Val Ser Ser Ala Lys Thr Lys
   115 120
<210> 3
   <211> 213
   <212> PRT
   <213> Homo sapiens
<400> 3
   Gln Ile Val Leu Ser Gln Ser Pro Ala Ile Leu Ser Ala Ser Pro Gly
   1 5 10 15
   Glu Lys Val Thr Met Thr Cys Arg Ala Ser Ser Ser Val Ser Tyr Ile
               20                  25                  30
   His Trp Tyr Gln Gln Lys Pro Gly Ser Ser Pro Lys Pro Trp Ile Tyr
           35                  40                  45
   Ala Thr Ser His Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Thr Ala Thr Tyr Tyr Cys Gln Gln Trp Ser Ser Asn Pro Phe Thr
                   85                  90                  95
   Phe Gly Ser Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 4
   <211> 102
   <212> PRT
   <213> Homo sapiens
<400> 4
   Gln Ile Val Leu Ser Gln Ser Pro Ala Ile Leu Ser Ala Ser Pro Gly
   1               5                   10                  15
   Glu Lys Val Thr Met Thr Cys Arg Ala Ser Ser Ser Val Ser Tyr Ile
   20 25 30
   His Trp Tyr Gln Gln Lys Pro Gly Ser Ser Pro Lys Pro Trp Ile Tyr
   35 40 45
   Ala Thr Ser His Leu Ala Ser Gly Val Pro Ala Arg Phe Ser Gly Ser
   50 55 60
   Gly Ser Gly Thr Ser Tyr Ser Leu Thr Ile Ser Arg Val Glu Ala Glu
   65 70 75 80
   Asp Thr Ala Thr Tyr Tyr Cys Gln Gln Trp Ser Ser Asn Pro Phe Thr
   85 90 95
   Phe Gly Ser Gly Thr Lys
   100
<210> 5
   <211> 450
   <212> PRT
   <213> Homo sapiens
<400> 5
   Gln Val Gln Leu Gln Gln Ser Gly Ala Glu Leu Ala Arg Pro Gly Ala
   1 5 10 15
   Ser Val Lys Met Ser Cys Lys Ala Ser Gly Tyr Thr Phe Thr Thr Tyr
   20 25 30
   Thr Met His Trp Val Lys Gln Arg Pro Gly Gln Gly Leu Glu Trp Ile
   35 40 45
   Gly Tyr Ile Asn Pro Ser Ser Gly Tyr Thr Asn Tyr Asn Gln Lys Phe
   50 55 60
   Lys Asp Lys Ala Thr Leu Thr Ala Asp Lys Ser Ser Ser Thr Ala Ser
   65 70 75 80
   Met Gln Leu Ser Ser Leu Thr Ser Glu Asp Ser Ala Val Tyr Tyr Cys
   85 90 95
   Ala Arg Glu Arg Ala Val Leu Val Pro Tyr Ala Met Asp Tyr Trp Gly
   100 105 110
   Gln Gly Thr Ser Val Thr Val Ser Ser Ala Lys Thr Lys Gly Pro Ser
   115 120 125
   Val Phe Pro Leu Ala Pro Cys Ser Arg Ser Thr Ser Glu Ser Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Lys Arg Val Glu Ser Lys Tyr Gly
       210                 215                 220
   Pro Pro Cys Pro Pro Cys Pro Ala Pro Glu Phe Glu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Gln Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu Gly Lys
   435 440 445
   Ala Ser
   450
<210> 6
   <211> 125
   <212> PRT
   <213> Homo sapiens
<400> 6
   Gln Val Gln Leu Gln Gln Ser Gly Ala Glu Leu Ala Arg Pro Gly Ala
   1 5 10 15
   Ser Val Lys Met Ser Cys Lys Ala Ser Gly Tyr Thr Phe Thr Thr Tyr
   20 25 30
   Thr Met His Trp Val Lys Gln Arg Pro Gly Gln Gly Leu Glu Trp Ile
   35 40 45
   Gly Tyr Ile Asn Pro Ser Ser Gly Tyr Thr Asn Tyr Asn Gln Lys Phe
   50 55 60
   Lys Asp Lys Ala Thr Leu Thr Ala Asp Lys Ser Ser Ser Thr Ala Ser
   65 70 75 80
   Met Gln Leu Ser Ser Leu Thr Ser Glu Asp Ser Ala Val Tyr Tyr Cys
   85 90 95
   Ala Arg Glu Arg Ala Val Leu Val Pro Tyr Ala Met Asp Tyr Trp Gly
   100 105 110
   Gln Gly Thr Ser Val Thr Val Ser Ser Ala Lys Thr Lys
   115 120 125
<210> 7
   <211> 213
   <212> PRT
   <213> Homo sapiens
<400> 7
   Gln Ile Val Leu Thr Gln Ser Pro Ala Val Met Ser Ala Ser Pro Gly
   1               5                   10                  15
   Glu Lys Val Thr Ile Thr Cys Thr Ala Ser Ser Ser Leu Ser Tyr Met
               20                  25                  30
   His Trp Phe Gln Gln Lys Pro Gly Thr Ser Pro Lys Leu Trp Leu Tyr
           35                  40                  45
   Ser Thr Ser Ile Leu Ala Ser Gly Val Pro Thr Arg Phe Ser Gly Ser
       50                  55                  60
   Gly Ser Gly Thr Ser Tyr Ser Leu Thr Ile Ser Arg Met Glu Ala Glu
   65                  70                  75                  80
   Asp Ala Ala Thr Tyr Tyr Cys Gln Gln Arg Ser Ser Ser Pro Phe Thr
                   85                  90                  95
   Phe Gly Ser Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 8
   <211> 102
   <212> PRT
   <213> Homo sapiens
<400> 8
   Gln Ile Val Leu Thr Gln Ser Pro Ala Val Met Ser Ala Ser Pro Gly
   1               5                   10                  15
   Glu Lys Val Thr Ile Thr Cys Thr Ala Ser Ser Ser Leu Ser Tyr Met
               20                  25                  30
   His Trp Phe Gln Gln Lys Pro Gly Thr Ser Pro Lys Leu Trp Leu Tyr
           35                  40                  45
   Ser Thr Ser Ile Leu Ala Ser Gly Val Pro Thr Arg Phe Ser Gly Ser
       50                  55                  60
   Gly Ser Gly Thr Ser Tyr Ser Leu Thr Ile Ser Arg Met Glu Ala Glu
   65                  70                  75                  80
   Asp Ala Ala Thr Tyr Tyr Cys Gln Gln Arg Ser Ser Ser Pro Phe Thr
                   85                  90                  95
   Phe Gly Ser Gly Thr Lys
               100
<210> 9
   <211> 448
   <212> PRT
   <213> Homo sapiens
<400> 9
   Glu Val Gln Leu Gln Gln Ser Gly Pro Glu Leu Glu Lys Pro Gly Ala
   1               5                   10                  15
   Ser Val Lys Ile Ser Cys Lys Ala Ser Gly Tyr Ser Phe Thr Gly Tyr
               20                  25                  30
   Asn Met Asn Trp Val Lys Gln Ser Asn Gly Lys Ser Leu Glu Trp Ile
           35                  40                  45
   Gly Asn Ile Asp Pro Tyr Tyr Gly Asp Thr Asn Tyr Asn Gln Lys Phe
       50                  55                  60
   Lys Gly Lys Ala Thr Leu Thr Val Asp Lys Ser Ser Ser Thr Ala Tyr
   65                  70                  75                      80
   Met His Leu Lys Ser Leu Thr Ser Glu Asp Ser Ala Val Tyr Tyr Cys
                   85                  90                      95
   Ala Arg Pro Tyr Gly Ser Glu Ala Tyr Phe Ala Tyr Trp Gly Gln Gly
               100                 105                 110
   Thr Leu Val Thr Val Ser Ala Ala Lys Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Lys Thr Tyr Thr Cys Asn Val Asp His Lys Pro
           195                 200                 205
   Ser Asn Thr Lys Val Asp Lys Arg Val Glu Ser Lys Tyr Gly Pro Pro
       210                 215                 220
   Cys Pro Pro Cys Pro Ala Pro Glu Phe Glu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser Gln Glu Asp Pro Glu Val
               260                 265                 270
   Gln Phe Asn Trp Tyr Val Asp Gly Val Glu Val His Asn Ala Lys Thr
           275                 280                 285
   Lys Pro Arg Glu Glu Gln Phe Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Gly Leu Pro Ser Ser Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Gln Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Arg Leu Thr Val Asp Lys Ser Arg Trp
   405 410 415
   Gln Glu Gly Asn Val Phe Ser Cys Ser Val Met His Glu Ala Leu His
   420 425 430
   Asn His Tyr Thr Gln Lys Ser Leu Ser Leu Ser Leu Gly Lys Ala Ser
   435 440 445
<210> 10
   <211> 123
   <212> PRT
   <213> Homo sapiens
<400> 10
   Glu Val Gln Leu Gln Gln Ser Gly Pro Glu Leu Glu Lys Pro Gly Ala
   1 5 10 15
   Ser Val Lys Ile Ser Cys Lys Ala Ser Gly Tyr Ser Phe Thr Gly Tyr
   20 25 30
   Asn Met Asn Trp Val Lys Gln Ser Asn Gly Lys Ser Leu Glu Trp Ile
   35 40 45
   Gly Asn Ile Asp Pro Tyr Tyr Gly Asp Thr Asn Tyr Asn Gln Lys Phe
   50 55 60
   Lys Gly Lys Ala Thr Leu Thr Val Asp Lys Ser Ser Ser Thr Ala Tyr
   65 70 75 80
   Met His Leu Lys Ser Leu Thr Ser Glu Asp Ser Ala Val Tyr Tyr Cys
   85 90 95
   Ala Arg Pro Tyr Gly Ser Glu Ala Tyr Phe Ala Tyr Trp Gly Gln Gly
   100 105 110
   Thr Leu Val Thr Val Ser Ala Ala Lys Thr Lys
   115 120
<210> 11
   <211> 214
   <212> PRT
   <213> Homo sapiens
<400> 11
   Asp Ile Val Met Thr Gln Ser Pro Ala Thr Leu Ser Val Thr Pro Gly
   1 5 10 15
   Asp Arg Val Ser Leu Ser Cys Arg Ala Ser Gln Ser Ile Ser Asp Tyr
   20 25 30
   Leu His Trp Tyr Gln Gln Lys Ser His Glu Ser Pro Arg Leu Leu Ile
   35 40 45
   Lys Tyr Ala Ala Gln Ser Ile Ser Gly Ile Pro Ser Arg Phe Ser Gly
   50 55 60
   Ser Gly Ser Gly Ser Asp Phe Thr Leu Ser Ile Asn Gly Val Glu Pro
   65 70 75 80
   Glu Asp Val Gly Val Tyr Tyr Cys Gln Asn Gly His Ser Phe Pro Tyr
   85 90 95
   Thr Phe Gly Gly Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 12
   <211> 103
   <212> PRT
   <213> Homo sapiens
<400> 12
   Asp Ile Val Met Thr Gln Ser Pro Ala Thr Leu Ser Val Thr Pro Gly
   1               5                   10                  15
   Asp Arg Val Ser Leu Ser Cys Arg Ala Ser Gln Ser Ile Ser Asp Tyr
               20                  25                  30
   Leu His Trp Tyr Gln Gln Lys Ser His Glu Ser Pro Arg Leu Leu Ile
           35                  40                  45
   Lys Tyr Ala Ala Gln Ser Ile Ser Gly Ile Pro Ser Arg Phe Ser Gly
       50                  55                  60
   Ser Gly Ser Gly Ser Asp Phe Thr Leu Ser Ile Asn Gly Val Glu Pro
   65                  70                  75                  80
   Glu Asp Val Gly Val Tyr Tyr Cys Gln Asn Gly His Ser Phe Pro Tyr
                   85                  90                  95
   Thr Phe Gly Gly Gly Thr Lys
               100
<210> 13
   <211> 10
   <212> PRT
   <213> Homo sapiens
<400> 13
   Gly Phe Ser Leu Ser Asn Tyr Asp Ile Ser
   1               5                   10
<210> 14
   <211> 16
   <212> PRT
   <213> Homo sapiens
<400> 14
   Val Met Trp Thr Gly Gly Gly Ala Asn Tyr Asn Ser Ala Phe Met Ser
   1               5                   10                  15
<210> 15
   <211> 10
   <212> PRT
   <213> Homo sapiens
<400> 15
   Asp Ala Val Arg Tyr Trp Asn Phe Asp Val
   1 5 10
<210> 16
   <211> 10
   <212> PRT
   <213> Homo sapiens
<400> 16
   Arg Ala Ser Ser Ser Val Ser Tyr Ile His
   1 5 10
<210> 17
   <211> 7
   <212> PRT
   <213> Homo sapiens
<400> 17
   Ala Thr Ser His Leu Ala Ser
   1 5
<210> 18
   <211> 10
   <212> PRT
   <213> Homo sapiens
<400> 18
   Cys Gln Gln Trp Ser Ser Asn Pro Phe Thr
   1 5 10
<210> 19
   <211> 10
   <212> PRT
   <213> Homo sapiens
<400> 19
   Gly Tyr Thr Phe Thr Thr Tyr Thr Met His
   1 5 10
<210> 20
   <211> 17
   <212> PRT
   <213> Homo sapiens
<400> 20
   Tyr Ile Asn Pro Ser Ser Gly Tyr Thr Asn Tyr Asn Gln Lys Phe Lys
   1 5 10 15
   Asp
<210> 21
   <211> 12
   <212> PRT
   <213> Homo sapiens
<400> 21
   Glu Arg Ala Val Leu Val Pro Tyr Ala Met Asp Tyr
   1               5                   10
<210> 22
   <211> 10
   <212> PRT
   <213> Homo sapiens
<400> 22
   Thr Ala Ser Ser Ser Leu Ser Tyr Met His
   1               5                   10
<210> 23
   <211> 7
   <212> PRT
   <213> Homo sapiens
<400> 23
   Ser Thr Ser Ile Leu Ala Ser
   1               5
<210> 24
   <211> 9
   <212> PRT
   <213> Homo sapiens
<400> 24
   Gln Gln Arg Ser Ser Ser Pro Phe Thr
   1               5
<210> 25
   <211> 10
   <212> PRT
   <213> Homo sapiens
<400> 25
   Gly Tyr Ser Phe Thr Gly Tyr Asn Met Asn
   1               5                   10
<210> 26
   <211> 17
   <212> PRT
   <213> Homo sapiens
<400> 26
   Asn Ile Asp Pro Tyr Tyr Gly Asp Thr Asn Tyr Asn Gln Lys Phe Lys
   1               5                   10                  15
   Gly
<210> 27
   <211> 10
   <212> PRT
   <213> Homo sapiens
<400> 27
   Pro Tyr Gly Ser Glu Ala Tyr Phe Ala Tyr
   1               5                   10
<210> 28
   <211> 11
   <212> PRT
   <213> Homo sapiens
<400> 28
   Arg Ala Ser Gln Ser Ile Ser Asp Tyr Leu His
   1               5                   10
<210> 29
   <211> 7
   <212> PRT
   <213> Homo sapiens
<400> 29
   Tyr Ala Ala Gln Ser Ile Ser
   1               5
<210> 30
   <211> 9
   <212> PRT
   <213> Homo sapiens
<400> 30
   Gln Asn Gly His Ser Phe Pro Tyr Thr
   1               5

## Description

### BACKGROUND OF THE INVENTION

This application claims the benefit of priority to U.S. Provisional Patent Application Serial No. 62/006,575, filed June 2, 2014.

The invention was made with government support under Grant No. 1R21AI101810-01 awarded by the National Institutes of Health.

### 1. Field of the Invention

The present invention relates generally to the field of medicine. More particularly, it concerns pharmaceutical compositions for treating pathogenic or increased Th2 type cell responses in a subject in need thereof.

### 2. Background

Asthma and allergic diseases, such as allergic rhinitis (hay fever), food allergy, and atopic dermatitis (eczema), are common for all age groups in the United States. For example, asthma affects more than 17 million adults and more than 7 million children. Hay fever, respiratory allergies, and other allergies affect approximately 10 percent of children under 18 years old. In addition, food allergy affects an estimated 5 percent of children under 5 years old and 4 percent of children ages 5 to 17 years old and adults.

An allergy is a hypersensitivity disorder of the immune system. Symptoms include red eyes, itchiness, runny nose, eczema, hives, or an asthma attack. Allergies can play a major role in conditions such as asthma. In some people, severe allergies to environmental or dietary allergens or to medication may result in life-threatening reactions called anaphylaxis. Food allergies, and reactions to the venom of stinging insects such as wasps and bees are more often associated with these severe reactions. Not all reactions or intolerances are forms of allergy.

Allergic reactions occur when a person's immune system reacts to normally harmless substances in the environment. Allergen-induced pathogenic immune responses are the major causes of multiple types of allergic diseases, including allergic atopy and dermatitis, allergic rhinitis, and allergic asthma. The pathophysiology of such allergic immune disorders is complex and is often associated with several factors, e.g., genetic susceptibility, age, and route and dose of allergen exposure. Allergic reactions are distinctive because of excessive activation of certain white blood cells called mast cells and basophils by a type of antibody called Immunoglobulin E (IgE). This reaction results in an inflammatory response which can range from uncomfortable to dangerous.

Treatments for allergies include avoiding known allergens, steroids that non-specifically modify the immune system, and medications such as antihistamines and decongestants which reduce symptoms. Many of these medications are taken by mouth, although epinephrine, which is used to treat anaphylactic reactions, is injected. The use of non-specific immunosuppressants may alleviate allergic reactions, but may also compromise the host's immunity to pathogenic infections. Furthermore, medications such as antihistamines may be useful for alleviating symptoms of allergic responses, but may only work for a limited duration or for a subset of the population. Therefore, there is a need in the art for effective, specific therapies for the treatment of allergic responses.

US2012/231023 relates to a vaccine adjuvant based on targeting adjuvants to antibodies directly to antigen-presenting cells. US2013/052193 relates to controlling allergy and asthma by activating human DCS via dectin-1 or toll-like receptor 2 (TLR2). Ferwerda et al. (Cellular microbiology 10.10 (2008): 2058-2066) relates to Dectin-1 synergizing TLR2 and TLR4 for cytokine production in human primary monocytes and macrophages.

### SUMMARY OF THE INVENTION

This disclosure fulfills the aforementioned need in the art by providing therapeutic approaches with immune modifiers of the Th2 pathway for the treatment of allergic and inflammatory diseases. Aspects of the disclosure relate to a method for decreasing Th2-type cell responses in a subject in need thereof comprising administering to the subject a therapeutically effective amount of an anti-Dectin-1 antibody or antigen binding fragment thereof operatively linked to a TLR agonist.

The invention provides a therapeutically effective amount of an anti-Dectin-1 antibody or antigen binding fragment thereof operatively linked to Pam3CSK4 for use in a method for preventing or treating allergic and inflammatory disorders in a subject in need thereof comprising administering to the subject the therapeutically effective amount of the anti-Dectin-1 antibody or antigen binding fragment thereof operatively linked to Pam3CSK4.

The invention also provides a pharmaceutical composition comprising a therapeutically effective amount of an anti-Dectin-1 antibody or antigen binding fragment thereof operatively linked to Pam3CSK4.

The invention further provides a pharmaceutical composition of the invention for use in a method for: (i) decreasing Th2-type cell responses in a subject in need thereof; (ii) decreasing IgE levels in a subject in need thereof; or (iii) preventing or treating allergic disorders in a subject in need thereof, in which a therapeutically effective amount of said pharmaceutical composition is administered to the subject.

The term "operatively linked" refers to a situation where two components are combined to form the active complex prior to binding at the target site. For example, an antibody conjugated to one-half of a cohesin-dockerin complex and a TLR complexed to the other one-half of the cohesin-dockerin complex are operatively linked through complexation of the cohesin and dockerin molecules. The term operatively linked is also intended to refer to covalent or chemical linkages that conjugate two molecules together.

Also disclosed herein is a method for decreasing IgE levels in a subject in need thereof comprising administering to the subject a therapeutically effective amount of an anti-Dectin-1 antibody or antigen binding fragment thereof operatively linked to a TLR agonist.

Also disclosed herein is a method for preventing or treating allergic disorders in a subject in need thereof comprising administering to the subject a therapeutically effective amount of an anti-Dectin-1 antibody or antigen binding fragment thereof operatively linked to a TLR agonist. The allergic disorder may be one that is characterized as having an increased or a pathogenic Th2-type cell response or increased IgE level.

A TLR agonist may be one described herein or known in the art. A TLR agonist may be selected from a TLR2, TLR7, or a TLR8 agonist. A TLR agonist may be a TLR2 agonist. A TLR agonist may be Pam3CSK4. A TLR agonist may be a TLR7 or TLR8 agonist. A TLR7 or TLR8 agonist may be selected from ssRNA, and R848. A TLR agonist may be conjugated to the anti-Dectin-1 antibody or antigen binding fragment thereof. A TLR agonist may be chemically conjugated to the anti-Dectin-1 antibody or antigen binding fragment thereof.

Pam3CSK4 is a synthetic triacylated lipopeptide (LP) that mimics the acylated amino terminus of bacterial LPs. Pam3CSK4 is a potent activator of the proinflammatory transcription factor NF-κB. Activation is mediated by the interaction between TLR2 and TLR1 which recognize LPs with three fatty acids, a structural characteristic of bacterial LPs. The chemical name of Pam3CSK4 is N-Palmitoyl-S-[2,3-bis(palmitoyloxy)-(2RS)-propyl]-[R]-cysteinyl-[S]-seryl-[S]-lysyl-[S]-lysyl-[S]-lysyl-[S]-lysine. Pam3CSK4 is also sometimes referred to herein and in the art as "Pam3."

An antibody or antigen binding fragment may specifically bind to dectin-1 and activates cells via dectin-1. An antibody or antigen binding fragment thereof may bind to and activate human dectin-1. Dectin-1 is a protein that in humans is encoded by the CLEC7A gene. This gene encodes a member of the C-type lectin/C-type lectin-like domain (CTL/CTLD) superfamily. The encoded glycoprotein is a small type II membrane receptor with an extracellular C-type lectin-like domain fold and a cytoplasmic domain with an immunoreceptor tyrosine-based activation motif. It functions as a pattern-recognition receptor that recognizes a variety of beta-1,3-linked and beta-1,6-linked glucans from fungi and plants, and in this way plays a role in innate immune response. Expression is found on myeloid Dendritic cells, monocytes, macrophages and B cells. An antibody or antigen binding fragment may specifically bind and activate dectin-1 on an antigen presenting cell. The antigen presenting cell may be a dendritic cell. The dendritic cell may be in blood, peripheral blood, is a dermal dendritic cell, is a myeloid dendritic cell, is a dendritic cell that secretes IL-12, or is a mDC-1 cell. Dectin-1 is a transmembrane protein containing an immunoreceptor tyrosine-based activation (ITAM)-like motif in its intracellular tail (which is involved in cellular activation) and single C-type lectin like domain (carbohydrate-recognition domain, CRD) in the extracellular region (which recognized β-glucans and endogenous ligand on T cells). The CRD is separated from the membrane by a stalk region. CLEC7A contains putative N-linked sites of glycosylation in stalk region.

The Dectin-1 antibody conjugate or antigen binding fragment thereof may comprise an amino acid sequence that is at least or at most 70%, 75%, 80%, 85%, 90%, 95%, 96%, 97%, 98%, or 99% identical or similar to the Dectin-1 antibody or antigen binding fragment of any of SEQ ID NO:1-12 (or any range derivable therein). The Dectin-1 antibody conjugate or antigen binding fragment thereof may comprise a variable region comprising an amino acid sequence that is at least or at most 70%, 75%, 80%, 85%, 90%, 95%, 96%, 97%, 98%, or 99% (or any range derivable therein) identical or similar to the Dectin-1 antibody conjugate antibody or antigen binding fragment of any of SEQ ID NOs:2, 4, 6, 8, 10, and 12. The antibody may comprise a CDR having an amino acid sequence corresponding to any one of SEQ ID NOs:13-30. The Dectin-1 antibody conjugate or antigen binding fragment thereof may comprise a heavy or light chain amino acid sequence that is at least or at most 70%, 75%, 80%, 85%, 90%, 95%, 96%, 97%, 98%, or 99% (or any range derivable therein) identical or similar to the Dectin-1 antibody or antigen binding fragment of any of SEQ ID NOs:1, 3, 5, 7, 9, and 11. The antibody conjugate or antigen binding fragment thereof may comprise CDR1, CDR2, and/or CDR3 from the heavy and/or light chain variable region of a Dectin-1 antibody. The antibody conjugate or antigen binding fragment thereof may comprise a heavy chain comprising CDRs of SEQ ID NO:13-15, 19-21, or 25-27. The antibody conjugate or antigen binding fragment thereof may comprise a light chain comprising CDRs of SEQ ID NO:16-18, 22-24, or 28-30. The antibody conjugate or antigen binding fragment thereof may comprise all three CDRs from the light chain variable region and/or all three CDRs from the heavy chain variable region of a Dectin-1 antibody. The antibody conjugate or antigen binding fragment thereof may comprise a heavy chain comprising CDRs of SEQ ID NO:13-15 and a light chain comprising CDRs of SEQ ID NO:16-18. The antibody conjugate or antigen binding fragment thereof may comprise a heavy chain comprising CDRs of SEQ ID NO:19-21 and a light chain comprising CDRs of SEQ ID NO:22-24. The antibody conjugate or antigen binding fragment thereof may comprise a heavy chain comprising CDRs of SEQ ID NO:25-27 and a light chain comprising CDRs of SEQ ID NO:28-30.

The Dectin-1 antibody conjugate or antigen binding fragment or fragments described herein may include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more variant amino acids (or any range derivable therein) within at least, or at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 300, 400, 500, 550, 1000 or more contiguous amino acids, or any range derivable therein, of any of SEQ ID NOs:1-12.

The antibody may comprise a γ4 constant region. The γ4 constant region may comprise a substitution of glutamic acid for leucine at residue 235. The γ4 constant region may comprise a substitution of proline for serine at residue 228 in the hinge region.

In the methods described herein, the subject may be a human subject. The term "subject," "individual" or "patient" is used interchangeably herein and refers to a vertebrate, for example a primate, a mammal or preferably a human. Mammals may include equines, canines, bovines, ovines, murines, rats, simians, humans, farm animals, sport animals and pets.

The subject may be one that is suffering from or at risk from suffering from an allergic disorder or a Th2-mediated allergic disorder. The subject may be suffering from or at risk from suffering from an inflammatory disorder or a Th2-mediated inflammatory disorder. The Th2 response may be a Th2-mediated inflammatory response. The subject may exhibit one or more symptoms of the inflammatory disorder or has a history of suffering from the inflammatory disorder.

The Th2-mediated inflammatory disorder may be selected from such as asthma, chronic obstructive pulmonary disease, interstitial lung disease, chronic obstructive lung disease, chronic bronchitis, eosinophilic bronchitis, eosinophilic pneumonia, pneumonia, inflammatory bowel disease, atopic dermatitis, atopy, allergy, allergic rhinitis, idiopathic pulmonary fibrosis, scleroderma, emphysema, breast cancer, and ulcerative colitis. The Th2-mediated inflammatory disorder is breast cancer. The Th2-mediated inflammatory disorder may be ulcerative colitis. It is specifically contemplated that one or more of the listed Th2-mediated inflammatory disorders may be excluded.

Also disclosed herein are methods of treating a subject suffering from or at risk of developing type 1 diabetes. The compositions and antibody conjugates described herein may be used to treat inflammatory and/or Th2-mediated aspects of type 1 diabetes.

In some of the methods described herein, the administration may be performed prior to onset of an allergic and/or inflammatory reaction. The administration may be performed after onset of an allergic and/or inflammatory reaction.

The anti-Dectin-1 antibody or antigen binding fragment thereof operatively linked to a TLR agonist may be administered in an amount effective for the increase of one or more of Thl, Th17, and Treg cells in the subject. The Th2 cell responses may comprise CD4⁺ T cells.

The anti-Dectin-1 antibody or antigen binding fragment thereof operatively linked to a TLR agonist may be administered in a pharmaceutical composition. In certain aspects, the pharmaceutical composition does not contain an antigen or allergen. The pharmaceutical composition may consist essentially of an anti-dectin-1 antibody or antigen binding fragment thereof operatively linked to a TLR agonist. The antibody or antigen binding fragment thereof operatively linked to a TLR agonist may not be conjugated to an antigen or to a dockerin or cohesin molecule. The antibody or antigen binding fragment thereof operatively linked to a TLR agonist may not be covalently or operatively linked to an antigen or to a dockerin or to a cohesin molecule.

Also described herein are pharmaceutical compositions comprising the anti-Dectin-1 antibody or antigen binding fragment thereof operatively linked to a TLR agonist, as described above.

This disclosure also relates to an anti-Dectin-1 antibody or antigen binding fragment thereof operatively linked to a TLR agonist, as described herein, in the manufacture of a medicament for preventing or treating allergic disorders, for decreasing IgE levels, and/or for decreasing Th2-type cell responses in a subject in need thereof.

This disclosure also relates to the use of An anti-Dectin-1 antibody or antigen binding fragment thereof operatively linked to a TLR agonist, as described herein, for preventing or treating allergic disorders, for decreasing IgE levels, and/or for decreasing Th2-type cell responses in a subject in need thereof.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating certain embodiments of the invention, are given by way of illustration only. It is contemplated that any element specifically listed in the recited embodiments may also be specifically excluded from certain embodiments. For example, certain embodiments may relate to compositions comprising an antigen. Further embodiments relate to compositions and methods that do not include an antigen or administration of an antigen.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****:** Shown are exemplary methods of chemical conjugation of Pam3 to anti-Dectin-1 antibody. A linker is attached to pam3CSK4 to help increase solubility and to prevent crosslinking of multiple pam3 molecules. A phosphine group is added to the αDectin-1, which can then react with the free azide on the Pam3CSK3, thus creating a conjugate between the two compounds.
**FIG. 2A-B** - αDectin-1-pam3 has no loss of binding and relatively unchanged TLR2 activity. (FIG. 2A) Binding capacity of antibody and pam3 conjugates in PBMCs. (FIG. 2B) TLR2 reporter cells with titrated amounts of either αDectin-1, pam3 or αDectin-1-pam3.
**FIG. 3** shows that the anti-Dectin-1-Pam3 conjugate can efficiently activate antigen presenting cells, including mDCs.
**FIG. 4A-B** shows that αDectin-1-pam3 conjugate can decrease TSLP-induced OX40L expression on blood mDCs. mDCs were first purified from a buffy coat then cultured with 20 ng/mL TSLP and either 100 ng/mL pam3, 10 µg/mL of anti-dectin-1 or 10 µg/mL of αDectin-1-pam3. cells were harvested and stained after 48 hours. (FIG. 4A) mDC staining and (FIG. 4B) compiled results.
**FIG. 5** shows that the anti-Dectin-1-Pam3 conjugate treatment results in decreased Th2 type T cell responses.
**FIG. 6** shows the chromatogram and mass spectra of the PAM₃CSK₄CK-biotin product.
**FIG. 7** shows the chromatogram of the PAM3-biotin-DBCO product.
**FIG. 8A-B** shows that the addition of TLR2-L to Dectin-1 activation leads to decreased HA-1 specific Th2-type CD4+ T Cell responses. (FIG. 8A) CFSE-labeled CD4+ T cells were co-cultured for 7 days with DCs loaded with either αDectin-1-HA alone or αDectin-1-HA plus TLR2-L. (FIG. 8B) T cells were re-stimulated with HA1 peptides and Cytokine levels were analyzed by Luminex.
**Fig. 9A-B** - **αDectin-1-Pam3 activates cells in a titration-dependent manner. (****FIG. 9A****)** PBMCs and **(****FIG. 9B****)** mDCs were cultured for 24 to 48 hours, then supernatants were harvested for Luminex analysis.

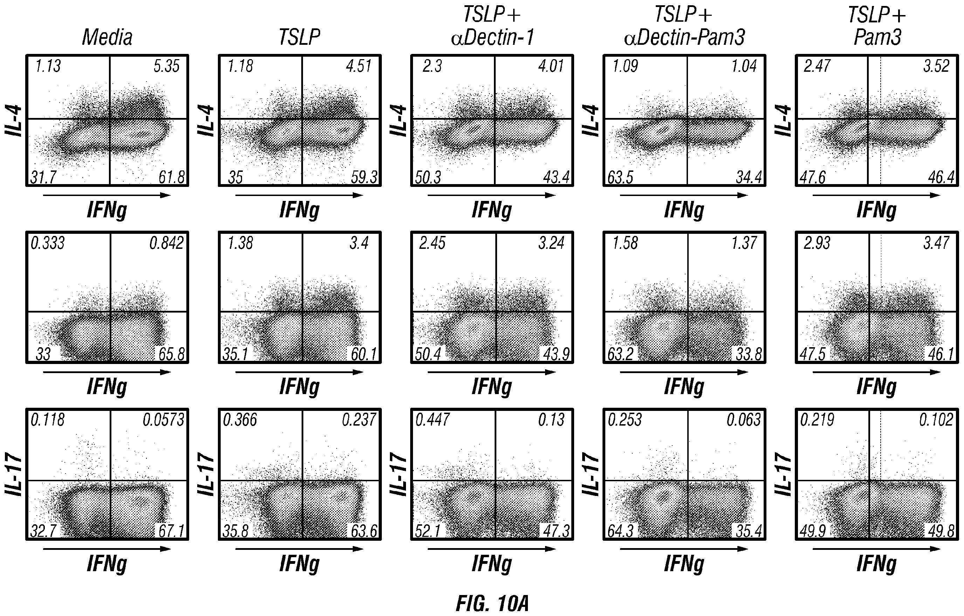

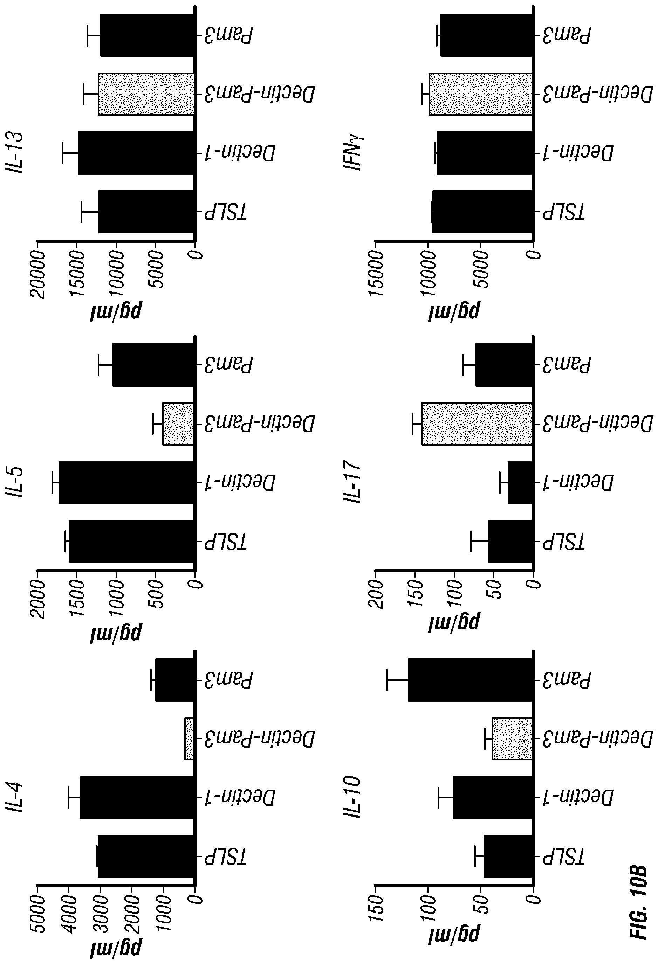

**Fig 10A-B** **- αDectin-1-pam3 conjugate can decrease TSLP-mDC induced T_{H}2-type CD4⁺ T cell responses while promoting T_{H}1- and T_{H}17-type CD4⁺ T cells** responses. mDCs were first primed with 40 ng/mL TSLP and either αdectin-1 or αDectin-1-pam3 at 20 ug/mL. After 24 hrs, naive CD4+ T cells are added to the mDCs and cultured for an additional 6 days. (FIG. 10A) Intracellular cytokine levels were analyzed by intracellular staining in cells stimulated with PMA/Ionomycin for 6 hours and with brefeldinA for 4 hrs. (FIG. 10B) Cell supernatant cytokine levels were measured by stimulating the cells with αCD3/CD28 beads for 48 hrs.
**Fig 11A-B** **- αDectin-1-pam3 treatment decreases HDMA-specific serum IgE in NHP in vivo.** (FIG. 11A) NHP model for atopy was generated by sensitizing the animals to HDMA. (FIG. 11B) HDMA-specific serum IgE levels. The arrows represent when αDectin-1-pam3 was given.
**FIG. 12** **- DCs activated with curdlan result in decreased antigen-specific TH2** responses. Antigen (Flu HA1) and Curdlan were incubated with dendritic cells and CD4+ T cells, followed by restimulation with HA-1-derived peptides. The flu HA1-specific CD4+ T cell responses such as IFNγ, IL-4, IL-5, and IL-13.
**FIG. 13** - **Curdlan downregulates total TH2 responses.** Anti-DC receptor antigen (Flu HA1) was incubated with dendritic cells and CD4+ T cells, followed by restimulation with PMA/lonomycin. Cells were then immunostained for IL-13 and IL-5. The percentage of IL-13+ cells decreased significantly in curdlan-treated cultures.
**FIG. 14** shows the intracellular cytokine staining data from the serum of the NHP model of Atopy depicted in FIG. 11A.

### DESCRIPTION OF ILLUSTRATIVE EMBODIMENTS

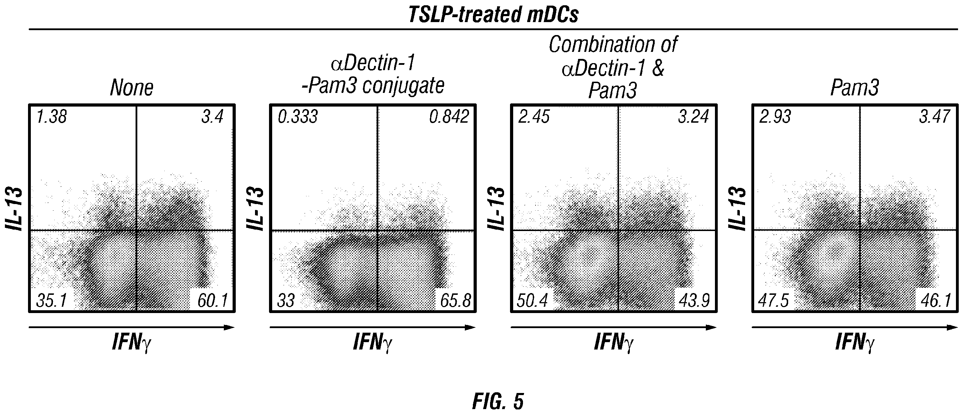

Methods and compositions described herein can be used to treat inflammatory and allergic disorders. It was discovered that administration of anti-dectin-1 antibodies operatively linked to a TLR agonist are useful to control allergen-specific Th2-type immune responses. As shown in **FIG. 5****,** anti-dectin-1 conjugated to Pam3 reduced Th2 type T cell responses whereas the unconjugated counterpart composition with anti-dectin-1 and Pam3 failed to reduce Th2 type T cell responses. Without being limited to any scientific theory, it is believed that such antibody conjugates have the ability to target specific subsets of cells (*i.e.* cells expressing dectin-1 and a TLR) and may lead to a lower effective concentration required to achieve a therapeutic effect as compared to non-conjugated counterparts. Furthermore, the ability to target specific subsets of cells may result in fewer undesired side effects or off-target effects compared to the non-conjugated counterparts.

### I. Antibodies

Methods and compositions of the disclosure relate to anti-dectin-1 antibodies and antibody binding fragments thereof operatively linked to a TLR agonist. As used herein, an "antibody" includes whole antibodies and any antigen binding fragment or a single chain thereof. Thus the term "antibody" includes any protein or peptide containing molecule that comprises at least a portion of an immunoglobulin molecule. Examples of such may include a complementarity determining region (CDR) of a heavy or light chain or a ligand binding portion thereof, a heavy chain or light chain variable region, a heavy chain or light chain constant region, a framework (FR) region or any portion thereof or at least one portion of a binding protein.

The antibody can be any of the various antibodies described herein, non-limiting, examples of such include a polyclonal antibody, a monoclonal antibody, a chimeric antibody, a recombinant antibody, a human antibody, a veneered antibody, a diabody, a humanized antibody, an antibody derivative, a recombinant humanized antibody, or a derivative or fragment of each thereof.

Antibodies can be generated using conventional techniques known in the art and are well-described in the literature. Several methodologies exist for production of polyclonal antibodies. For example, polyclonal antibodies are typically produced by immunization of a suitable mammal such as chickens, goats, guinea pigs, hamsters, horses, mice, rats, and rabbits. An antigen is injected into the mammal, induces the B-lymphocytes to produce immunoglobulins specific for the antigen. Immunoglobulins may be purified from the mammal's serum. Common variations of this methodology include modification of adjuvants, routes and site of administration, injection volumes per site and the number of sites per animal for optimal production and humane treatment of the animal. For example, adjuvants typically are used to improve or enhance an immune response to antigens. Most adjuvants provide for an injection site antigen depot, which allows for a slow release of antigen into draining lymph nodes. Other adjuvants include surfactants which promote concentration of protein antigen molecules over a large surface area and immunostimulatory molecules. Non-limiting examples of adjuvants for polyclonal antibody generation include Freund's adjuvants, Ribi adjuvant system, and Titermax. Polyclonal antibodies can be generated using methods known in the art some of which are described in U.S. Pat. Nos. 7,279,559; 7,119,179; 7,060,800; 6,709,659; 6,656,746; 6,322,788; 5,686,073; and 5,670,153.

Unless specified otherwise, the antibodies can be polyclonal or monoclonal and can be isolated from any suitable biological source, e.g., murine, rat, sheep or canine.

The antibody may be a monoclonal antibody. As used herein, "monoclonal antibody" refers to an antibody obtained from a substantially homogeneous antibody population. Monoclonal antibodies are highly specific, as each monoclonal antibody is directed against a single determinant on the antigen. The antibodies may be detectably labeled, e.g., with a radioisotope, an enzyme which generates a detectable product, or a fluorescent protein. The antibodies may be further conjugated to other moieties, such as members of specific binding pairs, e.g., biotin (member of biotin-avidin specific binding pair). The antibodies may also be bound to a solid support, for example to polystyrene plates or beads.

Monoclonal antibodies can be generated using conventional hybridoma techniques known in the art and well-described in the literature. For example, a hybridoma is produced by fusing a suitable immortal cell line (e.g., a myeloma cell line such as Sp2/0, Sp2/0-AG14, NSO, NS1, NS2, AE-1, L.5, P3X63Ag8,653, Sp2 SA3, Sp2 MAI, Sp2 SS1, Sp2 SA5, U397, MIA 144, ACT IV, MOLT4, DA-1, JURKAT, WEHI, K-562, COS, RAJI, NIH 313, HL-60, MLA 144, NAMAIWA, NEURO 2A, CHO, PerC.6, YB2/O) or the like, or heteromyelomas, fusion products thereof, or any cell or fusion cell derived there from, or any other suitable cell line as known in the art, with antibody producing cells, such as isolated or cloned spleen, peripheral blood, lymph, tonsil, or other immune or B cell containing cells, or any other cells expressing heavy or light chain constant or variable or framework or CDR sequences, either as endogenous or heterologous nucleic acid, as recombinant or endogenous, viral, bacterial, algal, prokaryotic, amphibian, insect, reptilian, fish, mammalian, rodent, equine, ovine, goat, sheep, primate, eukaryotic, genomic DNA, cDNA, rDNA, mitochondrial DNA or RNA, chloroplast DNA or RNA, hnRNA, mRNA, tRNA, single, double or triple stranded, hybridized or any combination thereof. Antibody producing cells can also be obtained from the peripheral blood or, preferably the spleen or lymph nodes, of humans or other suitable animals that have been immunized with the antigen of interest. Any other suitable host cell can also be used for expressing-heterologous or endogenous nucleic acid encoding an antibody, specified fragment or variant thereof. The fused cells (hybridomas) or recombinant cells can be isolated using selective culture conditions or other suitable known methods, and cloned by limiting dilution or cell sorting, or other known methods.

Other suitable methods of producing or isolating antibodies of the requisite specificity can be used, for example including methods that select recombinant antibody from a peptide or protein library (e,g., a bacteriophage, ribosome, oligonucleotide, cDNA, or the like, display library; e.g., as available from various commercial vendors such as MorphoSys (Martinsreid/Planegg, Del.), Biolnvent (Lund, Sweden), Affitech (Oslo, Norway) using methods known in the art. Art known methods are described in the patent literature some of which include U.S. Pat. Nos. 4,704,692; 5,723,323; 5,763,192; 5,814,476; 5,817,483; 5,824,514; 5,976,862. Alternative methods rely upon immunization of transgenic animals (e.g., SCID mice, Nguyen et al. (1977) Microbiol. Immunol. 41:901-907 (1997); Sandhu et al. (1996) Crit, Rev. Biotechnol. 16:95-118; Eren et al. (1998) Mumma 93:154-161 that are capable of producing a repertoire of human antibodies, as known in the art and/or as described herein. Such techniques may include ribosome display Wanes et al. (1997) Proc. Natl. Acad. Sci. USA, 94:4937-4942; Hanes et al, (1998) Proc. Natl. Acad. Sci. USA 95:14130-14135); single cell antibody producing technologies (e,g., selected lymphocyte antibody method ("SLAM") (U.S. Pat. No. 5,627,052, Wen et al, (1987) J. Immunol 17:887-892; Babcook et al. (1996) Proc. Natl. Acad. Sci. USA 93:7843-7848); gel microdroplet and flow cytometry (Powell et al. (1990) Biotechnol. 8:333-337; One Cell Systems, (Cambridge, Mass).; Gray et al. (1995) J. Imm. Meth. 182:155-163; and Kenny et al, (1995) Bio. Technol. 13:787-790); B-cell selection (Steenbakkers et al. (1994) Molec. Biol. Reports 19:125-134).

The terms "polyclonal antibody" or "polyclonal antibody composition" as used herein refer to a preparation of antibodies that are derived from different B-cell lines. They are a mixture of immunoglobulin molecules secreted against a specific antigen, each recognizing a different epitope.

The term "mouse antibody" as used herein, is intended to include antibodies having variable and constant regions derived from mouse germline immunoglobulin sequences.

As used herein, chimeric antibodies are antibodies whose light and heavy chain genes have been constructed, typically by genetic engineering, from antibody variable and constant region genes belonging to different species. The antibody may be a mouse/human chimeric antibody.

The antibody may comprise a modification and is an "antibody derivative." The term "antibody derivative" includes post-translational modification to linear polypeptide sequence of the antibody or fragment. For example, U.S. Pat. No. 6,602,684 B1 describes a method for the generation of modified glycol-forms of antibodies, including whole antibody molecules, antibody fragments, or fusion proteins that include a region equivalent to the Fc region of an immunoglobulin, having enhanced Fe-mediated cellular toxicity, and glycoproteins so generated.

The antibodies provided herein also include derivatives that are modified by the covalent attachment of any type of molecule to the antibody such that covalent attachment does not prevent the antibody from generating an anti-idiotypic response. Antibody derivatives may include antibodies that have been modified by glycosylation, acetylation, pegylation, phosphorylation, amidation, derivatization by known protecting/blocking groups, proteolytic cleavage, linkage to a cellular ligand or other protein, etc. Additionally, the derivatives may contain one or more non-classical amino acids.

Antibody derivatives can also be prepared by delivering a polynucleotide encoding an antibody to a suitable host such as to provide transgenic animals or mammals, such as goats, cows, horses, sheep that produce such antibodies in their milk. These methods are known in the art and are described for example in U.S. Pat. Nos. 5,827,690; 5,849,992; 4,873,316; 5,849,992; 5,994,616; 5,565,362; and 5,304,489.

Antibody derivatives also can be prepared by delivering a polynucleotide to provide transgenic plants and cultured plant cells (e.g., tobacco, maize, and duckweed) that produce such antibodies, specified portions or variants in the plant parts or in cells cultured therefrom. Antibody derivatives have also been produced in large amounts from transgenic plant seeds including antibody fragments, such as single chain antibodies (scFv's), including tobacco seeds and potato tubers. See, e.g., Conrad et al. (1998) Plant Mol. Biol. 38:101-109 and references cited therein. Thus, antibodies can also be produced using transgenic plants, according to know methods.

Antibody derivatives also can be produced, for example, by adding exogenous sequences to modify immunogenicity or reduce, enhance or modify binding, affinity, on-rate, off-rate, avidity, specificity, half-life, or any other suitable characteristic. Generally part or all of the non-human or human CDR sequences are maintained while the non-human sequences of the variable and constant regions are replaced with human or other amino acids.

The tem "variable region" refers to a portion of the antibody that gives the antibody its specificity for binding antigen. The variable region is typically located at the ends of the heavy and light chains. Variable loops of β-strands, three each on the light (VL) and heavy (VH) chains are responsible for binding to the antigen. These loops are referred to as the "complementarity determining regions" (CDRs).

In general, the CDR residues are directly and most substantially involved in influencing antigen binding. Humanization or engineering of antibodies can be performed using any known method such as those described in U.S. Pat. Nos. 5,723,323; 5,976,862; 5,824,514; 5,817,483; 5,814,476; 5,763,192; 5,723,323; 5,766,886; 5,714,352; 6,204,023; 6,180,370; 5,693,762; 5,530,101; 5,585,089; 5,225,539; and 4,816,567.

The term "constant region" refers to a portion of the antibody that is identical in all antibodies of the same isotype. The constant region differs in antibodies of different isotypes.

The antibody may be a humanized antibody. As used herein, the term "humanized antibody" or "humanized immunoglobulin" refers to a human/non-human chimeric antibody that contains a minimal sequence derived from non-human immunoglobulin. For the most part, humanized antibodies are human immunoglobulins (recipient antibody) in which residues from a variable region of the recipient are replaced by residues from a variable region of a non-human species (donor antibody) such as mouse, rat, rabbit, or non-human primate having the desired specificity, affinity and capacity. Humanized antibodies may comprise residues that are not found in the recipient antibody or in the donor antibody. The humanized antibody can optionally also comprise at least a portion of an immunoglobulin constant region (Fc), typically that of a human immunoglobulin, a non-human antibody containing one or more amino acids in a framework region, a constant region or a CDR, that have been substituted with a correspondingly positioned amino acid from a human antibody. In general, humanized antibodies are expected to produce a reduced immune response in a human host, as compared to a non-humanized version of the same antibody. The humanized antibodies may have conservative amino acid substitutions which have substantially no effect on antigen binding or other antibody functions. Conservative substitutions groupings include:glycine-alanine, valine-leucine-isoleucine, phenylalanine-tyrosine, lysine-arginine, alanine-valine, serine-threonine and asparagine-glutamine.

Chimeric, humanized or primatized antibodies can be prepared based on the sequence of a reference monoclonal antibody prepared using standard molecular biology techniques. DNA encoding the heavy and light chain immunoglobulins can be obtained from the hybridoma of interest and engineered to contain non-reference (e.g., human) immunoglobulin sequences using standard molecular biology techniques. For example, to create a chimeric antibody, the murine variable regions can be linked to human constant regions using methods known in the art (U.S. Pat. No. 4,816,567). To create a humanized antibody, the murine CDR regions can be inserted into a human framework using methods known in the art (U.S. Pat. No. 5,225,539 and U.S. Pat. Nos. 5,530,101; 5,585,089; 5,693,762 and 6,180,370). Similarly, to create a primatized antibody the murine CDR regions can be inserted into a primate framework using methods known in the art (WO 93/02108 and WO 99/55369). Methods of determining CDRs from the sequence of a variable region are known in the art (see, for example, Zhao and Lu, "A germline knowledge based computational approach for determining antibody complementarity determining regions." Mol. Immunol., (2010) 47(4):694-700).

Techniques for making partially to fully human antibodies are known in the art and any such techniques can be used. Fully human antibody sequences may be made in a transgenic mouse which has been engineered to express human heavy and light chain antibody genes. Multiple strains of such transgenic mice have been made which can produce different classes of antibodies. B cells from transgenic mice which are producing a desirable antibody can be fused to make hybridoma cell lines for continuous production of the desired antibody. (See for example, Russel et al. (2000) Infection and Immunity April 2000:1820-1826; Gallo et al. (2000) European J. of Immun. 30:534-540; Green (1999) J. of Immun. Methods 231:11-23; Yang et al. (1999A) J. of Leukocyte Biology 66:401-410; Yang (1999B) Cancer Research 59(6):1236-1243; Jakobovits (1998) Advanced Drug Reviews 31:33-42; Green and Jakobovits (1998) J. Exp. Med. 188(3):483-495; Jakobovits (1998) Exp. Opin. Invest. Drugs 7(4):607-614; Tsuda et al. (1997) Genomics 42:413-421; Sherman-Gold (1997) Genetic Engineering News 17(14); Mendez et al. (1997) Nature Genetics 15:146-156; Jakobovits (1996) Weir's Handbook of Experimental Immunology, The Integrated Immune System Vol. IV, 194.1-194.7; Jakobovits (1995) Current Opinion in Biotechnology 6:561-566; Mendez et al, (1995) Genomics 26:294-307; Jakobovits (1994) Current Biology 4(8):761-763; Arbones et al. (1994) :Immunity 1(4):247-260; Jakobovits (1993) Nature 362(6417):255-258; Jakobovits et al. (1993) Proc. Natl. Acad. Sci. USA 90(6):2551-2555; and U.S. Pat. No. 6,075,181.)

Antibodies also can be modified to create chimeric antibodies. Chimeric antibodies are those in which the various domains of the antibodies' heavy and light chains are coded for by DNA from more than one species. See, e.g., U.S. Pat. No. 4,816,567.

Alternatively, antibodies can also be modified to create veneered antibodies. Veneered antibodies are those in which the exterior amino acid residues of the antibody of one species are judiciously replaced or "veneered" with those of a second species so that the antibodies of the first species will not be immunogenic in the second species thereby reducing the immunogenicity of the antibody. Since the antigenicity of a protein is primarily dependent on the nature of its surface, the immunogenicity of an antibody could be reduced by replacing the exposed residues which differ from those usually found in another mammalian species antibodies. This judicious replacement of exterior residues should have little, or no, effect on the interior domains, or on the interdomain contacts. Thus, ligand binding properties should be unaffected as a consequence of alterations which are limited to the variable region framework residues. The process is referred to as "veneering" since only the outer surface or skin of the antibody is altered, the supporting residues remain undisturbed.

The procedure for "veneering" makes use of the available sequence data for human antibody variable domains compiled by Kabat et al. (1987) Sequences of Proteins of Immunological interest, 4th ed., Bethesda, Md., National Institutes of Health, updates to this database, and other accessible U.S. and foreign databases (both nucleic acid and protein). Non-limiting examples of the methods used to generate veneered antibodies include EP 519596; U.S. Pat. No. 6,797,492; and described in Padlan et al. (1991) Mol. Immunol. 28(4-5):489-498.

The term "antibody derivative" also includes "diabodies" which are small antibody fragments with two antigen-binding sites, wherein fragments comprise a heavy chain variable domain (VH) connected to a light chain variable domain (VL) in the same polypeptide chain. (See for example, EP 404,097; WO 93/11161; and Hollinger et al. (1993) Proc. Natl. Acad. Sci. USA 90:6444-6448.) By using a linker that is too short to allow pairing between the two domains on the same chain, the domains are forced to pair with the complementary domains of another chain and create two antigen-binding sites. (See also, U.S. Pat. No. 6,632,926 to Chen et al, which discloses antibody variants that have one or more amino acids inserted into a hypervariable region of the parent antibody and a binding affinity for a target antigen which is at least about two fold stronger than the binding affinity of the parent antibody for the antigen).

The term "antibody derivative" further includes engineered antibody molecules, fragments and single domains such as scFv, dAbs, nanobodies, minibodies, Unibodies, and Affibodies & Hudson (2005) Nature Biotech 23(9):1126-36; U.S. Patent Publication US 2006/0211088; PCT Publication WO2007/059782; U.S. Pat. No. 5,831,012).

The term "antibody derivative" further includes "linear antibodies". The procedure for making linear antibodies is known in the art and described in Zapata et al. (1995) Protein Eng. 8(10):1057-1062. Briefly, these antibodies comprise a pair of tandem Ed segments (V.sub.H-C.sub.H 1-VH-C.sub.H1) which form a pair of antigen binding regions. Linear antibodies can be bispecific or monospecific.

Antibodies can be recovered and purified from recombinant cell cultures by known methods such as protein A purification, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography ("HPLC") can also be used for purification.

If an antibody being tested binds with protein or polypeptide, then the antibody being tested and the antibodies are equivalent. An equivalent may be one that binds dectin-1 and provides the same activity such as the stimulation of DC cells to secrete IL-10, the increased production of antigen-specific T regulatory cells, and/or the suppression of allogeneic or pathogenic T cell responses.

It also is possible to determine without undue experimentation, whether an antibody has the same specificity as antibodies contemplated herein by determining whether the antibody being tested prevents an antibody from binding the protein or polypeptide with which the antibody is normally reactive. If the antibody being tested competes with an antibody as shown by a decrease in binding by the monoclonal antibody, then it is likely that the two antibodies bind to the same or a closely related epitope. Alternatively, one can pre-incubate an antibody for use with a protein with which it is normally reactive, and determine if the antibody being tested is inhibited in its ability to bind the antigen. If the antibody being tested is inhibited then, in all likelihood, it has the same, or a closely related, epitopic specificity as the antibody.

The term "antibody" also is intended to include antibodies of all immunoglobulin isotypes and subclasses unless specified otherwise. An isotype refers to the genetic variations or differences in the constant regions of the heavy and light chains of an antibody. In humans, there are five heavy chain isotypes: IgA, IgD, IgG, IgE, and IgM and two light chain isotypes: kappa and lambda. The IgG class is divided into four isotypes: IgG1, IgG2, IgG3 and IgG4 in humans, and IgG1, IgG2a, IgG2b and IgG3 in mice. They share more than 95% homology in the amino acid sequences of the Fc regions but show major differences in the amino acid composition and structure of the hinge region. Particular isotypes of a monoclonal antibody can be prepared either directly by selecting from an initial fusion, or prepared secondarily, from a parental hybridoma secreting a monoclonal antibody of different isotype by using the sib selection technique to isolate class switch variants using the procedure described in Steplewski et al. (1985) Proc. Natl. Acad. Sci. USA 82:8653 or Spira et al, (1984) J. Immunol. Methods 74:307. Alternatively, recombinant DNA techniques may be used.

The isolation of other monoclonal antibodies with the specificity of the monoclonal antibodies described herein can also be accomplished by one of ordinary skill in the art by producing anti-idiotypic antibodies. Herlyn et al. (1986) Science 232:100. An anti-idiotypic antibody is an antibody which recognizes unique determinants present on the monoclonal antibody of interest.

In some aspects, it will be useful to detectably or therapeutically label the antibody. Methods for conjugating antibodies to these agents are known in the art. For the purpose of illustration only, antibodies can be labeled with a detectable moiety such as a radioactive atom, a chromophore, a fluorophore, or the like. Such labeled antibodies can be used for diagnostic techniques, either in vivo, or in an isolated test sample.

The antibody or antigen binding fragment may further comprise a modification. The modification may be a conservative amino acid mutation within the VH and/or VL CDR 1, CDR 2 and/or CDR 3 regions, of conservative amino acid mutations in the Fc hinge region, pegylation, conjugation to a serum protein, conjugation to human serum albumin, conjugation to a detectable label, conjugation to a diagnostic agent, conjugation to an enzyme, conjugation to a fluorescent, luminescent, or bioluminescent material, conjugation to a radioactive material, or conjugation to a therapeutic agent.

As used herein, the term "label" intends a directly or indirectly detectable compound or composition that is conjugated directly or indirectly to the composition to be detected, e.g., polynucleotide or protein such as an antibody so as to generate a "labeled" composition. The term also includes sequences conjugated to the polynucleotide that will provide a signal upon expression of the inserted sequences, such as green fluorescent protein (GFP). The label may be detectable by itself (e.g. radioisotope labels or fluorescent labels) or, in the case of an enzymatic label, may catalyze chemical alteration of a substrate compound or composition which is detectable. The labels can be suitable for small scale detection or more suitable for high-throughput screening. As such, suitable labels may include radioisotopes, fluorochromes, chemiluminescent compounds, dyes, and proteins, including enzymes. The label may be simply detected or it may be quantified. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator) component.

Examples of luminescent labels that produce signals may include bioluminescence and chemiluminescence. Detectable luminescence response generally comprises a change in, or an occurrence of, a luminescence signal. Suitable methods and luminophores for luminescently labeling assay components are known in the art and described for example in Haugland, Richard P. (1996) Handbook of Fluorescent Probes and Research Chemicals (6.sup.th ed.). Examples of luminescent probes may include aequorin and luciferases.

Examples of suitable fluorescent labels may include fluorescein, rhodamine, tetramethylrhodamine, eosin, erythrosin, coumarin, methyl-coumarins, pyrene, Malacite green, stilbene, Lucifer Yellow, Cascade Blue.TM., and Texas Red. Other suitable optical dyes are described in the Haugland, Richard P. (1996) Handbook of Fluorescent Probes and Research Chemicals (6.sup.th ed.).

In another aspect, the fluorescent label is functionalized to facilitate covalent attachment to a cellular component present in or on the surface of the cell or tissue such as a cell surface marker. Suitable functional groups, including, but not are limited to, isothiocyanate groups, amino groups, haloacetyl groups, maleimides, succinimidyl esters, and sulfonyl halides, all of which may be used to attach the fluorescent label to a second molecule. The choice of the functional group of the fluorescent label will depend on the site of attachment to either a linker, the agent, the marker, or the second labeling agent.

Attachment of the fluorescent label may be either directly to the cellular component or compound or alternatively, can by via a linker. Suitable binding pairs for use in indirectly linking the fluorescent label to the intermediate may include antigens/antibodies, e.g., rhodamine/anti-rhodamine, biotin/avidin and biotin/strepavidin.

The coupling of antibodies to low molecular weight haptens can increase the sensitivity of the antibody in an assay. The haptens can then be specifically detected by means of a second reaction. For example, it is common to use haptens such as biotin, which reacts avidin, or dinitrophenol, pyridoxal, and fluorescein, which can react with specific antihapten antibodies. See, Harlow and Lane (1988) supra.

The variable region of an antibody can be modified by mutating amino acid residues within the VH and/or VL CDR 1, CDR 2 and/or CDR 3 regions to improve one or more binding properties (e.g., affinity) of the antibody. Mutations may be introduced by site-directed mutagenesis or PCR-mediated mutagenesis and the effect on antibody binding, or other functional property of interest, can be evaluated in appropriate in vitro or in vivo assays. Preferably conservative modifications are introduced and typically no more than one, two, three, four or five residues within a CDR region are altered. The mutations may be amino acid substitutions, additions or deletions.

Framework modifications can be made to the antibodies to decrease immunogenicity, for example, by "backmutating" one or more framework residues to the corresponding germline sequence.

In addition, an antibody may be engineered to include modifications within the Fc region to alter one or more functional properties of the antibody, such as serum half-fife, complement fixation, Fc receptor binding, and/or antigen-dependent cellular cytotoxicity. Such modifications may include alterations of the number of cysteine residues in the hinge region to facilitate assembly of the light and heavy chains or to increase or decrease the stability of the antibody (U.S. Pat. No. 5,677,425) and amino acid mutations in the Fc hinge region to decrease the biological half life of the antibody (U.S. Pat. No. 6,165,745).

Additionally, one or more antibodies may be chemically modified. Glycosylation of an antibody can be altered, for example, by modifying one or more sites of glycosylation within the antibody sequence to increase the affinity of the antibody for antigen (U.S. Pat. Nos. 5,714,350 and 6,350,861). Alternatively, to increase antibody-dependent cell-mediated cytotoxicity, a hypofucosylated antibody having reduced amounts of fucosyl residues or an antibody having increased bisecting GlcNac structures can be obtained by expressing the antibody in a host cell.sub.-- with altered glycosylation mechanism (Shields, R. L. et al., 2002 J. Biol. Chem. 277:26733-26740; Umana et al., 1999 Nat. Biotech. 17:176-180).

Antibodies can be pegylated to increase biological half-life by reacting the antibody or fragment thereof with polyethylene glycol (PEG) or a reactive ester or aldehyde derivative of PEG, under conditions in which one or more PEG groups become attached to the antibody or antibody fragment. Antibody pegylation may be carried out by an acylation reaction or an alkylation reaction with a reactive PEG molecule (or an analogous reactive watersoluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. The antibody to be pegylated can be an aglycosylated antibody. Methods for pegylating proteins are known in the art and can be applied to one or more antibodies(EP 0 154 316 and EP 0 401 384).

Additionally, antibodies may be chemically modified by conjugating or fusing the antigen-binding region of the antibody to serum protein, such as human serum albumin, to increase half-life of the resulting molecule. Such approach is for example described in EP 0322094 and EP 0 486 525.

The antibodies or fragments thereof may be conjugated to a diagnostic agent and used diagnostically, for example, to monitor the development or progression of a disease and determine the efficacy of a given treatment regimen. Examples of diagnostic agents include enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody or fragment thereof, or indirectly, through a 1 inker using techniques known in the art. Examples of suitable enzymes include horseradish peroxidase, alkaline phosphatase, beta-galactosidase, or acetylcholinesterase. Examples of suitable prosthetic group complexes include streptavidin/biotin and avidin/biotin. Examples of suitable fluorescent materials include umbelliferone, fluorescein, fluorescein isothiocyanate, rhodamine, dichlorotriazinylamine fluorescein, dansyl chloride or phycoerythrin. An example of a luminescent material includes luminol. Examples of bioluminescent materials include luciferase, luciferin, and aequorin. Examples of suitable radioactive material include .sup.125I, .sup.131I, Indium-111, Lutetium-171, Bismuth-212, Bismuth-213, Astatine-211, Copper-62, Copper-64, Copper-67, Yttrium-90, Iodine-125, Iodine-131, Phosphorus-32, Phosphorus-33, Scandium-47, Silver-111, Gallium-67, Praseodymium-142, Samarium-153, Terbium-161, Dysprosium-166, Holmium-166, Rhenium-186, Ithenium-188, Rhenium-189, Lead-212, Radium-223, Actinium-225, Iron-59, Selenium-75, Arsenic-77, Strontium-89, Molybdenum-99, Rhodium-1105, Palladium-109, Praseodymium-143, Promethium-149, Erbium-169, Iridium-194, Gold-198, Gold-199, and Lead-211. Monoclonal antibodies may be indirectly conjugated with radiometal ions through the use of bifunctional chelating agents that are covalently linked to the antibodies. Chelating agents may be attached through amities (Meares et al., 1984 Anal. Biochem. 142: 68-78); sulfhydral groups (Koyama 1994 Chem. Abstr. 120: 217262t) of amino acid residues and carbohydrate groups (Rodwell et al. 1986 PNAS USA 83: 2632-2636; Quadri et al. 1993 Nucl. Med. Biol. 20: 559-570).

Additional suitable conjugated molecules include ribonuclease (RNase), DNase I, an antisense nucleic acid, an inhibitory RNA molecule such as a siRNA molecule, an immunostimulatory nucleic acid, aptamers, ribozymes, triplex forming molecules, and external guide sequences. Aptamers are small nucleic acids ranging from 15-50 bases in length that fold into defined secondary and tertiary structures, such as stern-loops or G-quartets, and can bind small molecules, such as ATP (U.S. Pat. No. 5,631,146) and theophilline (U.S. Pat. No. 5,580,737), as well as large molecules, such as reverse transcriptase (U.S. Pat. No. 5,786,462) and thrombin (U.S. Pat. No. 5,543,293). Ribozymes are nucleic acid molecules that are capable of catalyzing a chemical reaction, either intramolecularly or intermolecularly. Ribozymes typically cleave nucleic acid substrates through recognition and binding of the target substrate with subsequent cleavage. Triplex forming function nucleic acid molecules can interact with double-stranded or single-stranded nucleic acid by forming a triplex, in which three strands of DNA form a complex dependant on both Watson-Crick and Hoogsteen base-pairing. Triplex molecules can bind target regions with high affinity and specificity.

The functional nucleic acid molecules may act as effectors, inhibitors, modulators, and stimulators of a specific activity possessed by a target molecule, or the functional nucleic acid molecules may possess a de novo activity independent of any other molecules. The antibody may be a stimulator of dendritic cells

The conjugated agents can be linked to the antibody directly or indirectly, using any of a large number of available methods. For example, an agent can be attached at the hinge region of the reduced antibody component via disulfide bond formation, using crosslinkers such as N-succinyl 3-(2-pyridyldithio)proprionate (SPDP), or via a carbohydrate moiety in the Fc region of the antibody (Yu et al. 1994 Int. J. Cancer 56: 244; Upeslacis et al., "Modification of Antibodies by Chemical Methods," in Monoclonal antibodies: principles and applications, Birch et al. (eds.), pages 187-230 (Wiley-Liss, Inc. 1995); Price, "Production and Characterization of Synthetic Peptide-Derived Antibodies," in Monoclonal antibodies: Production, engineering and clinical application, Ritter et al. (eds.), pages 60-84 (Cambridge University Press 1995)).

Techniques for conjugating agents to antibodies are well known (Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al, (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeted Antibody in Cancer Therapy", in Monoclonal Antibodies For Cancer Detection And Therapy, Baldwin et al. (eds.), pp. 303-16 (Academic Press 1985), and Thorpe et al., The Preparation And Cytotoxic Properties Of Antibody-Toxin Conjugates" 1982 Immunol. Rev. 62:119-58),

Antibodies or antigen-binding regions thereof can be linked to another functional molecule such as another antibody or ligand for a receptor to generate a bi-specific or multi-specific molecule that binds to at least two or more different binding sites or target molecules. Linking of the antibody to one or more other binding molecules, such as another antibody, antibody fragment, peptide or binding mimetic, can be done, for example, by chemical coupling, genetic fusion, or noncovalent association. Multi-specific molecules can further include a third binding specificity, in addition to the first and second target epitope.

Bi-specific and multi-specific molecules can be prepared using methods known in the art. For example, each binding unit of the hi-specific molecule can be generated separately and then conjugated to one another. When the binding molecules are proteins or peptides, a variety of coupling or cross-linking agents can be used for covalent conjugation. Examples of cross-linking agents include protein A, carbodiimide, N-succinimidyl-S-acetylthioacetate (SATA), 5,5'-dithiobis(2-nitroberizoic acid) (DTNB), o-phenylenedimaleimide (oRDM), N-succinimidyl-3-(2-pyridyldithio)propionate (SPDP), and sulfosuccinimidyl 4-(N-maleimidomethyl)cyclohaxane-I-carboxylate (sulfo-SMCC) (Karpovsky et al., 1984 J. Exp. Med. 160:1686; Liu et al., 1985 Proc. Natl. Acad. Sci. USA 82:8648). When the binding molecules are antibodies, they can be conjugated by sulfhydryl bonding of the C-terminus hinge regions of the two heavy chains.

The antibodies or fragments thereof may be linked to a moiety that is toxic to a cell to which the antibody is bound to form "depleting" antibodies. These antibodies are particularly useful in applications where it is desired to deplete an NK cell.

The antibodies may also be attached to solid supports, which are particularly useful for immunoassays or purification of the target antigen. Such solid supports may include glass, cellulose, polyacrylamide, nylon, polystyrene, polyvinyl chloride or polypropylene.

The antibodies also can be bound to many different carriers. Thus, compositions are also provided containing the antibodies and another substance, active or inert. Examples of well-known carriers include glass, polystyrene, polypropylene, polyethylene, dextran, nylon, amylase, natural and modified cellulose, polyacrylamide, agarose, and magnetite. The nature of the carrier may be either soluble or insoluble. Those skilled in the art will know of other suitable carriers for binding monoclonal antibodies, or will be able to ascertain such, using routine experimentation.

### Constructs

The sequences given below, when presented as antibody H or L chain or protein secreted by mammalian cells are shown as amino acids without signal peptide (i.e., as 'mature' secreted protein), while the DNA sequences are the entire coding region including signal sequences if present.

All examples of H chain constructs are typically used in co-transfection of CHO cells with matching L chain vectors. Also, immunotherapeutics may have humanized variable regions.

manti-Dectin-1-11B6.4-H-V-hIgG4H-C]; SEQ ID NO:1:

The above sequence is a chimera between the H chain variable region of the mAb 11B6.4 and the C region of hIgG4.

The H chain variable region of the mAb 11B6.4 is shown in SEQ ID NO:2:

The CDRs of the H chain variable region of the mAb 11B6.4 are: GFSLSNYDIS (SEQ ID NO:13), VMWTGGGANYNSAFMS (SEQ ID NO:14), and DAVRYWNFDV (SEQ ID NO:15).

[manti-Dectin-1-11B6.4-K-LV-hIgGK-C] is the corresponding L chain chimera; SEQ ID NO:3:

The L chain variable region of the manti-Dectin-1-11B6.4-K-LV-hIgGK-C is shown in SEQ ID NO:4:

The CDRs of the L chain variable region of the manti-Dectin-1-11B6.4-K-LV-hIgGK-C are: RASSSVSYIH (SEQ ID NO:16), ATSHLAS (SEQ ID NO:17), and CQQWSSNPFT (SEQ ID NO:18).

manti-Dectin-1-15E2.5-H-V-hIgG4H-C]; SEQ ID NO:5:

The above sequence is a chimera between the H chain variable region of the mAb 15E2.5 and the C region of hIgG4.

The H chain variable region of the mAb 15E2.5 is shown in SEQ ID NO:6:

The CDRs of the H chain variable region of the mAb 15E2.5 are: GYTFTTYTMH (SEQ ID NO:19), YINPSSGYTNYNQKFKD (SEQ ID NO:20), and ERAVLVPYAMDY (SEQ ID NO:21).

[manti-Dectin-1-15E2.5-K-V-hIgGK-C] is the corresponding L chain chimera; SEQ ID NO:7:

The L chain variable region of the manti-Dectin-1-15E2.5-K-V-hIgGK-C is shown in SEQ ID NO:8:

The CDRs of the L chain variable region of the manti-Dectin-1-15E2.5-K-V-hIgGK-C are: TASSSLSYMH (SEQ ID NO:22), STSILAS (SEQ ID NO:23), and QQRSSSPFT (SEQ ID NO:24).

manti-Dectin-1-2D8.2D4-H-V-hIgG4H-C]; SEQ ID NO:9:

The above sequence is a chimera between the H chain variable region of the mAb 2D8.2D4 and the C region of hIgG4.

The H chain variable region of the mAb 2D8.2D4 is shown in SEQ ID NO:10:

The CDRs of the H chain variable region of the mAb 2D8.2D4 are: GYSFTGYNMN (SEQ ID NO:25), NIDPYYGDTNYNQKFKG (SEQ ID NO:26), and PYGSEAYFAY (SEQ ID NO:27).

[manti-Dectin-1-2D8.2D4-K-V-hIgGK-C] is the corresponding L chain chimera; SEQ ID NO:11:

The L chain variable region of the manti-Dectin-1-2D8.2D4-K-V-hIgGK-C is shown in SEQ ID NO:12:

The CDRs of the H chain variable region of the mAb 2D8.2D4 are: RASQSISDYLH (SEQ ID NO:28), YAAQSIS (SEQ ID NO:29), and QNGHSFPYT (SEQ ID NO:30).

### II. TLR agonist

TLR agonists are known in the art. TLR agonists may include an agonist to TLR1 (*e.g.* peptidoglycan or triacyl lipoproteins), TLR2 (*e.g.* lipoteichoic acid; peptidoglycan from *Bacillus subtilis, E. coli* 0111:B4, *Escherichia coli* K12, or *Staphylococcus aureus;* atypical lipopolysaccharide (LPS) such as *Leptospirosis* LPS and *Porphyromonas gingivalis* LPS; a synthetic diacylated lipoprotein such as FSL-1 or Pam2CSK4; lipoarabinomannan or lipomannan from *M. smegmatis;* triacylated lipoproteins such as Pam3CSK4; lipoproteins such as MALP-2 and MALP-404 from mycoplasma; *Borrelia burgdorferi* OspA; Porin from *Neisseria meningitidis* or *Haemophilus influenza; Yersinia* LcrV; lipomannan from *Mycobacterium* or *Mycobacterium tuberculosis; Trypanosoma cruzi* GPI anchor; *Schistosoma mansoni* lysophosphatidylserine; *Leishmania major* lipophosphoglycan (LPG); *Plasmodium falciparum* glycophosphatidylinositol (GPI); zymosan), TLR3 *(e.g.* double-stranded RNA, polyadenylic-polyuridylic acid (Poly(A:U)); polyinosine-polycytidylic acid (Poly(I:C)); polyinosine-polycytidylic acid high molecular weight (Poly(I:C) HMW); and polyinosine-polycytidylic acid low molecular weight (Poly(I:C) LMW)), TLR4 (*e.g.* LPS from *Escherichia coli* and *Salmonella* species); TLR5 *(e.g.* Flagellin from *B. subtilis, P. aeruginosa, or S. typhimurium*), TLR8 (*e.g.* single stranded RNAs such as ssRNA with 6UUAU repeats, RNA homopolymer (ssPolyU naked), HIV-1 LTR-derived ssRNA (ssRNA40), or ssRNA with 2 GUCCUUCAA repeats (ssRNA-DR)), TLR7 (*e.g.* imidazoquinoline compound imiquimod, Imiquimod VacciGrade™, Gardiquimod VacciGrade™, or Gardiquimod™; adenine analog CL264; base analog CL307; guanosine analog loxoribine; TLR7/8 (e.g. thiazoquinoline compound CL075; imidazoquinoline compound CLO97, R848, or R848 VacciGrade™), TLR9 (*e.g.* CpG ODNs); and TLR11 (*e.g. Toxoplasma gondii* Profilin). A TLR agonist may be a specific agonist listed above. A TLR agonist may be one that agonizes either one TLR or two TLRs specifically. A TLR may be a TLR2 agonist listed above.

A TLR may be selected from lipoteichoic acid; peptidoglycan from *Bacillus subtilis, E. coli* 0111:B4, *Escherichia coli* K12, or *Staphylococcus aureus;* atypical lipopolysaccharide (LPS) such as *Leptospirosis* LPS and *Porphyromonas gingivalis* LPS; a synthetic diacylated lipoprotein such as FSL-1 or Pam2CSK4; lipoarabinomannan or lipomannan from *M. smegmatis;* triacylated lipoproteins such as Pam3CSK4; lipoproteins such as MALP-2 and MALP-404 from mycoplasma; *Borrelia burgdorferi* OspA; Porin from *Neisseria meningitidis* or *Haemophilus influenza; Yersinia* LcrV; lipomannan from *Mycobacterium* or *Mycobacterium tuberculosis; Trypanosoma cruzi* GPI anchor; *Schistosoma mansoni* lysophosphatidylserine; *Leishmania major* lipophosphoglycan (LPG); *Plasmodium falciparum* glycophosphatidylinositol (GPI); and zymosan.

A TLR may be selected from *Porphyromonas gingivalis* LPS, Pam3CSK4, and peptidoglycan from *Bacillus subtilis, E. coli* 0111:B4, *Escherichia coli* K12, or *Staphylococcus aureus.*

A TLR may be selected from *Porphyromonas gingivalis* LPS and Pam3CSK4. A TLR may be Pam3CSK4.

### III. Pharmaceutical Compositions

Also disclosed herein are methods for treating allergic and/or inflammatory responses. They include compositions that can be used to induce or modify an immune response against an allergen or antigen e.g., a polypeptide, a peptide, a carbohydrate, a lipid or other molecule or molecular fragment and against developing a condition or disease caused by such an autoimmune response.

Administration of the compositions will typically be via any common route. This may include parenteral, orthotopic, intradermal, subcutaneous, intramuscular, intraperitoneal, intranasal, or intravenous injection. A vaccine composition may be inhaled (e.g., U.S. Pat. No. 6,651,655). Additional formulations which are suitable for other modes of administration include oral formulations. Oral formulations include such normally employed excipients as, for example, pharmaceutical grades of mannitol, lactose, starch, magnesium stearate, sodium saccharine, cellulose, magnesium carbonate. These compositions take the form of solutions, suspensions, tablets, pills, capsules, sustained release formulations or powders and contain about 10% to about 95% of active ingredient, preferably about 25% to about 70%.

Typically, compositions are administered in a manner compatible with the dosage formulation, and in such amount as will be therapeutically effective and immune modifying. The quantity to be administered depends on the subject to be treated. Precise amounts of active ingredient required to be administered depend on the judgment of the practitioner.

The manner of application may be varied widely. Any of the conventional methods for administration of an antibody are applicable. These are believed to include oral application on a solid physiologically acceptable base or in a physiologically acceptable dispersion, parenterally, e.g. by injection. The dosage of the pharmaceutical composition will depend on the route of administration and will vary according to the size and health of the subject.

In many instances, it will be desirable to have multiple administrations of at most about or at least about 3, 4, 5, 6, 7, 8, 9, 10 or more. The administrations may range from 2 day to twelve week intervals, more usually from one to two week intervals. The course of the administrations may be followed by assays for alloreactive immune responses and T cell activity.

The phrases "pharmaceutically acceptable" or "pharmacologically acceptable" refer to molecular entities and compositions that do not produce an adverse, allergic, or other untoward reaction when administered to an animal, or human. As used herein, "pharmaceutically acceptable carrier" includes any and all solvents, dispersion media, coatings, antibacterial and antifungal agents, isotonic and absorption delaying agents, etc. The use of such media and agents for pharmaceutical active substances is well known in the art. Except insofar as any conventional media or agent is incompatible with the active ingredients, its use in immunogenic and therapeutic compositions is contemplated.

The antibodies or antigen binding fragments can be formulated for parenteral administration, e.g., formulated for injection via the intravenous, intradermal, intramuscular, sub-cutaneous, or even intraperitoneal routes. A composition may be administered by intradermal injection. A composition may be administered by intravenous injection. The preparation of an aqueous composition that contains an anti-dectin-1 antibody or antigen binding fragment operatively linked to a TLR agonist that modifies the subject's immune condition will be known to those of skill in the art in light of the present disclosure. Typically, such compositions can be prepared as injectables, either as liquid solutions or suspensions; solid forms suitable for use to prepare solutions or suspensions upon the addition of a liquid prior to injection can also be prepared; and, the preparations can also be emulsified.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The compositions may be formulated into a neutral or salt form. Pharmaceutically acceptable salts, include the acid addition salts (formed with the free amino groups of the protein) and which are formed with inorganic acids such as, for example, hydrochloric or phosphoric acids, or organic acids such as acetic, oxalic, tartaric, and mandelic. Salts formed with the free carboxyl groups can also be derived from inorganic bases such as, for example, sodium, potassium, ammonium, calcium, or ferric hydroxides, and such organic bases as isopropylamine, trimethylamine, histidine and procaine.

The carrier can also be a solvent or dispersion medium containing, for example, water, ethanol, polyol (for example, glycerol, propylene glycol, and liquid polyethylene glycol), suitable mixtures thereof, and vegetable oils. The prevention of the action of microorganisms can be brought about by various antibacterial and antifungal agents, for example, parabens, chlorobutanol, phenol, sorbic acid, or thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active ingredients in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

An effective amount of therapeutic or prophylactic composition is determined based on the intended goal. The term "unit dose" or "dosage" refers to physically discrete units suitable for use in a subject, each unit containing a predetermined quantity of the composition calculated to produce the desired responses discussed above in association with its administration, i.e., the appropriate route and regimen. The quantity to be administered, both according to number of treatments and unit dose, depends on the result and/or protection desired. Precise amounts of the composition also depend on the judgment of the practitioner and are peculiar to each individual. Factors affecting dose include physical and clinical state of the subject, route of administration, intended goal of treatment (alleviation of symptoms versus cure), and potency, stability, and toxicity of the particular composition. Upon formulation, solutions will be administered in a manner compatible with the dosage formulation and in such amount as is therapeutically or prophylactically effective. The formulations are easily administered in a variety of dosage forms, such as the type of injectable solutions described above.

A pharmaceutical composition may comprise an antigen. A pharmaceutical composition may comprise an allergen. The allergen may be derived from dust mites. An anti-dectin-1 antibody or antigen binding fragment thereof operatively linked to a TLR agonist may be further operatively linked to an antigen or an allergen. The conjugation of the anti-Dectin-1 antibody to the antigen, allergen, or TLR may not be through a peptide bond. It is also specifically contemplated that embodiments of the disclosure include anti-dectin-1 antibody or antigen binding fragment thereof operatively linked to a TLR agonist without linkage to an antigen or without antigen in the pharmaceutical composition.

### IV. Combination Therapy

The compositions and related methods, particularly administration of an anti-dectin-1 antibody or antigen binding fragment operatively linked to a TLR agonist may also be used in combination with the administration of traditional therapies. These may include allergen immunotherapy, antihistamines, decongestants, anticholinergic nasal allergy sprays, steroid nasal sprays, allergy eye drops, leukotriene inhibitors, mast cell inhibitors, or allergy shots.

Antibody administration may precede or follow the other treatment by intervals ranging from minutes to weeks. Where the other agents are administered separately, one would generally ensure that a significant period of time did not expire between the time of each delivery, such that the agent and antibody would still be able to exert an advantageously combined effect on the subject. In such instances, it is contemplated that one may administer both modalities within about 12-24 h of each other and, more preferably, within about 6-12 h of each other. In some situations, it may be desirable to extend the time period for administration significantly, however, where several days (2, 3, 4, 5, 6 or 7) to several weeks (1, 2, 3, 4, 5, 6, 7 or 8) lapse between the respective administrations.

Administration of pharmaceutical compositions to a patient/subject will follow general protocols for the administration of such compounds, taking into account the toxicity, if any. It is expected that the treatment cycles would be repeated as necessary. It also is contemplated that various standard therapies, such as hydration, may be applied in combination with the described therapy.

### V. In Vitro or Ex Vivo Administration

As used herein, the term in vitro administration refers to manipulations performed on cells removed from or outside of a subject, e.g. cells in culture. The term ex vivo administration refers to cells which have been manipulated in vitro, and are subsequently administered to a subject. The term in vivo administration includes all manipulations performed within a subject, including administrations.

In certain aspects, the compositions may be administered either in vitro, ex vivo, or in vivo. In certain in vitro embodiments, autologous T cells are incubated with compositions described herein. The cells can then be used for in vitro analysis, or alternatively for ex vivo administration.

### VI. Therapeutic Applications

Also disclosed herein is the treatment for a disease or condition mediated by aberrant or elevated Th2-type cell responses. An anti-dectin-1 antibody or antigen binding fragment operatively linked to a TLR can be given to reduce or modify an immune response in a person having, suspected of having, or at risk of developing an allergic or inflammatory condition. In certain instances, the allergic or inflammatory condition is one that is associated with pathogenic Th2 type cell responses. Methods may be employed with respect to individuals who have tested positive for allergen reactivity or who are deemed to be at risk for developing such a condition or related condition.

Embodiments can be used to prevent, treat or ameliorate a number of allergic or inflammatory diseases. Non-limiting examples include asthma, type 1 diabetes, chronic obstructive pulmonary disease, interstitial lung disease, chronic obstructive lung disease, chronic bronchitis, eosinophilic bronchitis, eosinophilic pneumonia, pneumonia, inflammatory bowel disease, atopic dermatitis, atopy, allergy, allergic rhinitis, idiopathic pulmonary fibrosis, scleroderma, emphysema, breast cancer, and ulcerative colitis. Non-limiting examples of allergic disorders include allergic atopy and dermatitis, allergic rhinitis, allergic asthma, allergic responses to food (*e.g.* milk, egg, wheat, nut, fish, shellfish, sulfite, soy, and casein), environmental allergens (*e.g*. plant and animal allergens such as dander, dust mites, pollen, cedar, poison ivy, poison oak, poison sumac, etc...), insect bites (*e.g.* bee, wasp, yellow jacket, hornet, or fire ant stings), hay fever, allergic conjunctivitis, hives, mold, medication allergies (*e.g*. aspirin and penicillin), and cosmetic allergies.

### VII. Examples

The following examples are included to demonstrate certain embodiments. It should be appreciated by those of skill in the art that the techniques disclosed in the examples which follow represent techniques discovered by the inventor to function well in the practice of the invention, and thus can be considered to constitute preferred modes for its practice.

Allergen-induced pathogenic immune responses are the major causes of multiple types of allergic diseases, including allergic atopy and dermatitis, allergic rhinitis, and allergic asthma. The pathophysiology of such allergic immune disorders is complex and is often associated with several factors, e.g., genetic susceptibility, age, and route and dose of allergen exposure. Applicants hypothesize that therapeutic approaches with immune modifiers of the Th2 pathway represent a rational strategy for the treatment of such allergic diseases. However, current strategies targeting individual effector molecules (e.g., receptor antagonists and soluble receptors as well as neutralizing monoclonal antibodies (mAbs) to Th2 cytokines) may be insufficient to resolve the complex Th2-driven allergic immune disorders. Although specific immunotherapy (SIT) has been a hallmark of care among allergists for decades, considerable controversy still remains regarding its clinical efficacy, period of treatment, and socioeconomic consequences.

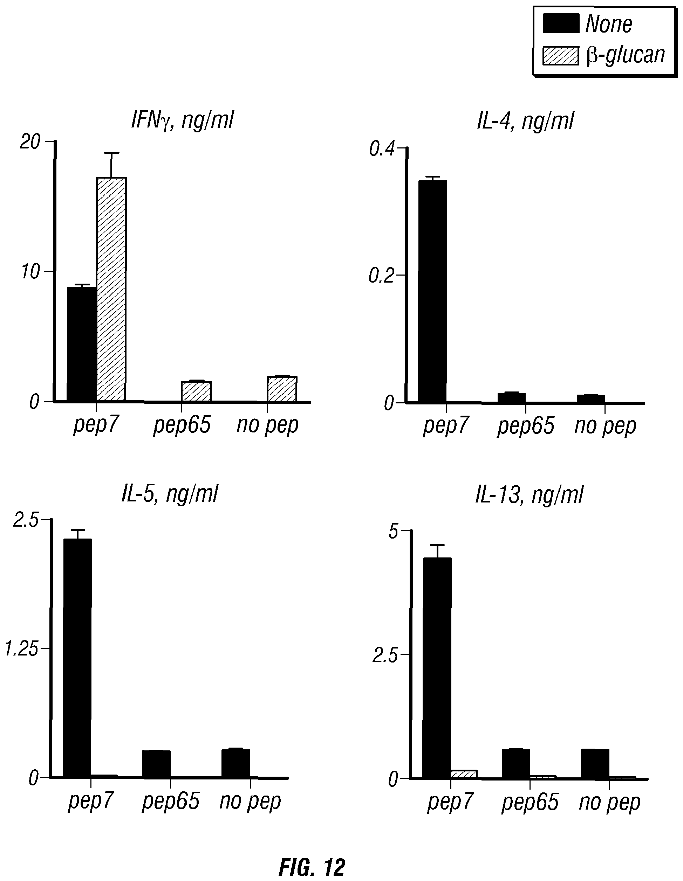

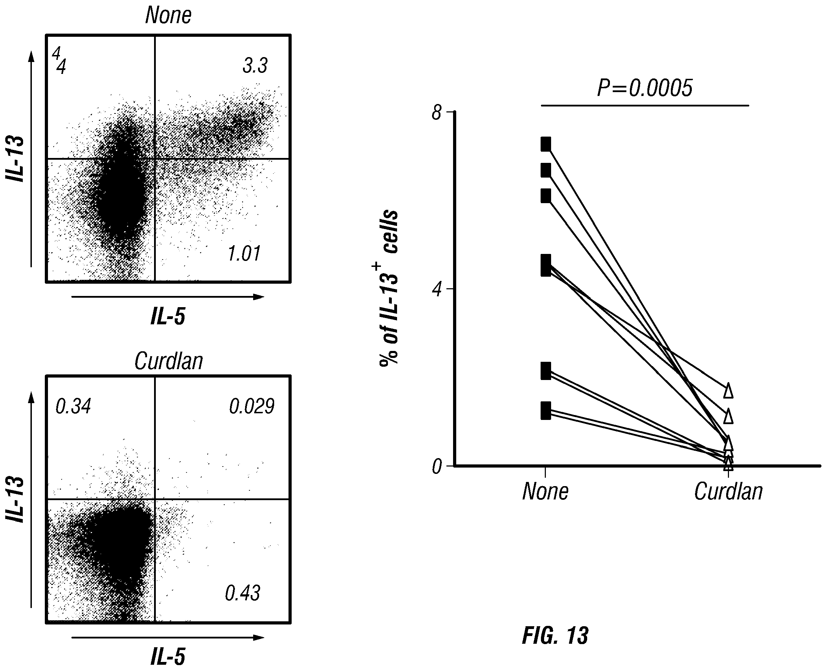

Thus, novel strategies that can effectively control allergen-specific Th2-type immune responses are required. Dendritic cells (DCs), major antigen presenting cells (APCs), can induce and control host immune responses by shaping the types of antigen-specific CD4⁺ T cells. In particular, Applicant's data has shown that human DCs activated via different lectin-like receptors (LLRs) can reprogram the quality and quantity of antigen-specific T cells in different ways. Of the numbers of LLRs tested, Dectin-1 shows a unique function of down-regulating Th2-type T cell responses. This applies to both memory and naive CD4⁺ T cells. Furthermore, treatment of allergic patient PBMCs with a Dectin-1 ligand (curdlan: β-glucan polymers extracted from *Aerobacterium*) results in significantly downregulated Th2-type T cell responses (FIGS. 12-13). Thus, it is hypothesized that targeting Dectin-1 and TLR expressed on APCs, especially DCs, will allow us to control allergen-specific Th2-type T cell responses followed by decreased IgE.

### Example 1: Anti-Dectin-1-Pam3 conjugate can suppress Th2 type inflammatory T cell responses

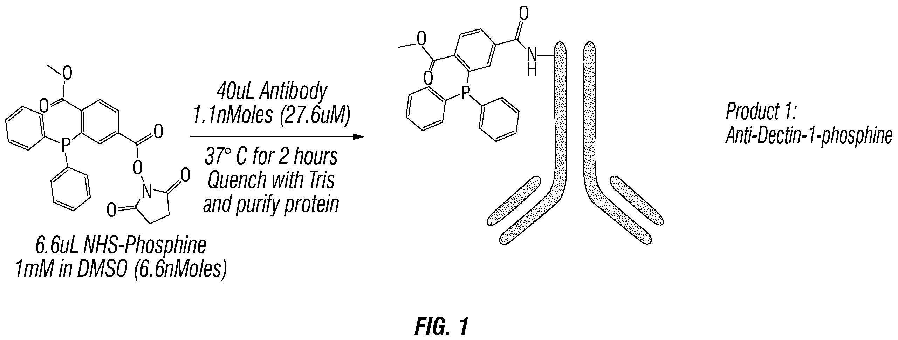

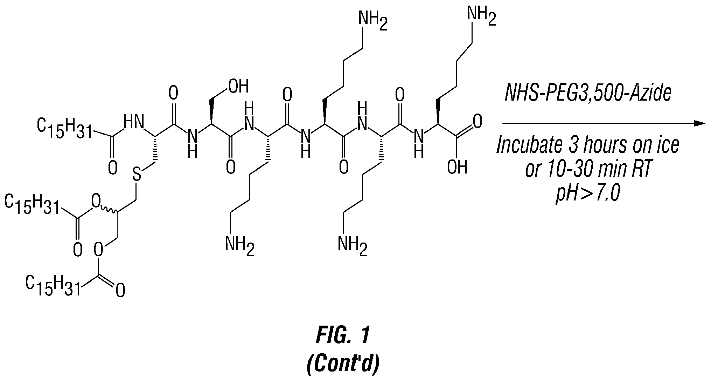

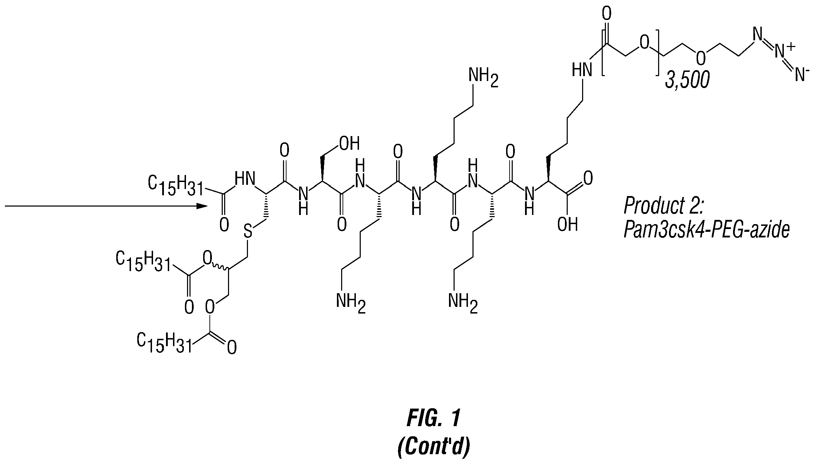

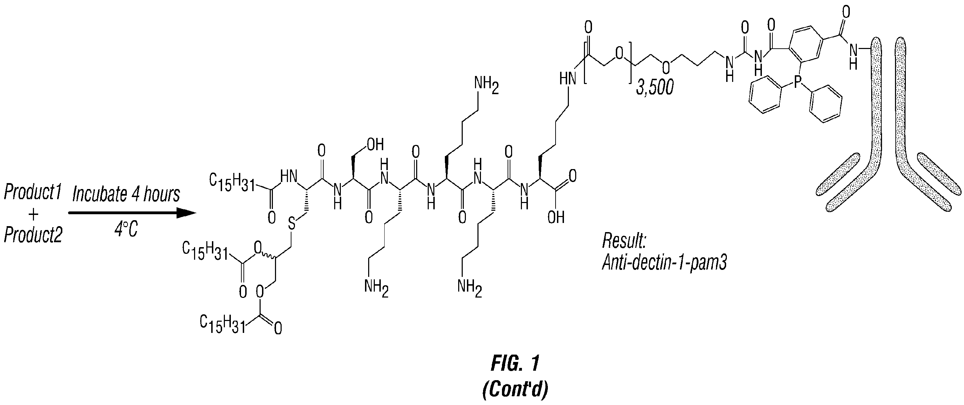

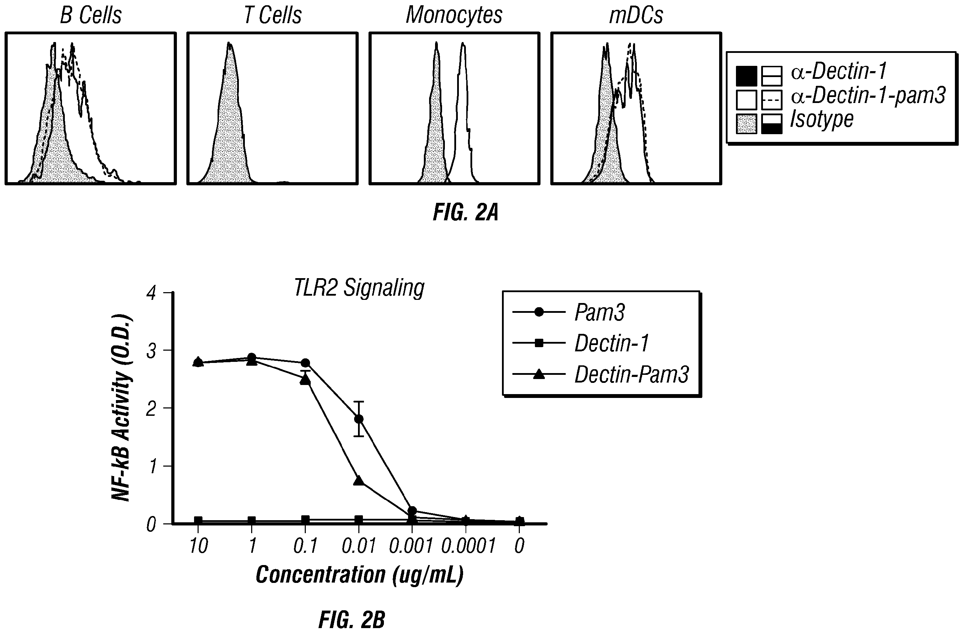

**Anti-Dectin-1-Pam3 conjugate binds to human antigen presenting cells.** To test and develop this therapeutic strategy, agonistic anti-human Dectin-1 mAb, which cross-reacts with Dectin-1 in non-human primates (NHP), was created. The antibody was conjugated to Pam3 (a.k.a. Pam3CSK4) according to **FIG. 1****.** PBMC of healthy donor were incubated for 20 min with 10 ug/ml of control antibody, anti-Dectin-1 antibody, and anti-Dectin-1-Pam3 conjugate at 4 C. Cells were washed and stained with goat anti-mouse IgG labeled with FITC. Cells were further stained with markers for B (CD19), T (CD3), monocytes (CD14), and myeloid dendritic cells (mDCs: Lin-HLA-DR+CD11c+CD123-). Binding of anti-Dectin-1 and anti-Dectin-1-Pam3 conjugate to different cell types were assessed by flow cytometry. As shown in **FIG. 2****,** anti-Dectin-1 and anti-Dectin-1-Pam3 conjugate equally bind to antigen presenting cells (B, monocytes, and mDCs), but not T cells which do not express Dectin-1. Taken together, our data demonstrate that anti-Dectin-1-Pam3 conjugate can efficiently target antigen presenting cells in human.

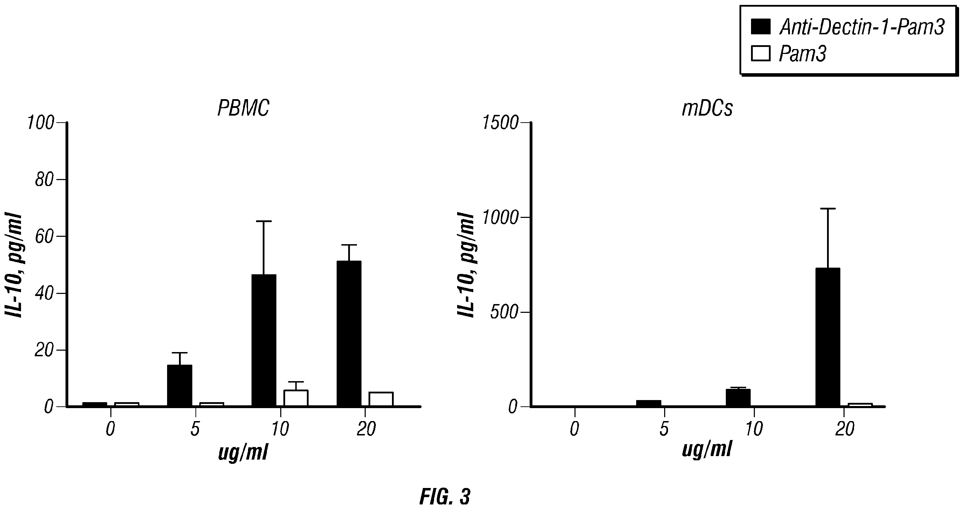

**Anti-Dectin-1-Pam3 conjugate is more potent than anti-Dectin-1 or Pam3 alone to activate mDCs and PBMC**. Next, the biological activity of anti-Dectin-1-Pam3 conjugate was compared with those of Pam3 and anti-Dectin-1 antibody alone **(****FIG. 3****).** 5x10⁵ PBMC and 2x10⁵ mDCs were incubated overnight in the presence of indicated concentrations of reagents and then the amount of IL-10 in the culture supernatants were assessed by ELISA. In both PBMC (left panel in **FIG. 3**) and mDC (right panel in **FIG. 3**) cultures, anti-Dectin-1-Pam3 conjugate was far more potent than Pam3 to induce IL-10 secretion. Soluble form of anti-Dectin-1 antibody alone did not induce PBMC or DCs to secrete IL-10 (data not shown). These results also indicate that anti-Dectin-1-Pam3 can efficiently deliver Pam3 to antigen presenting cells to stimulate them.

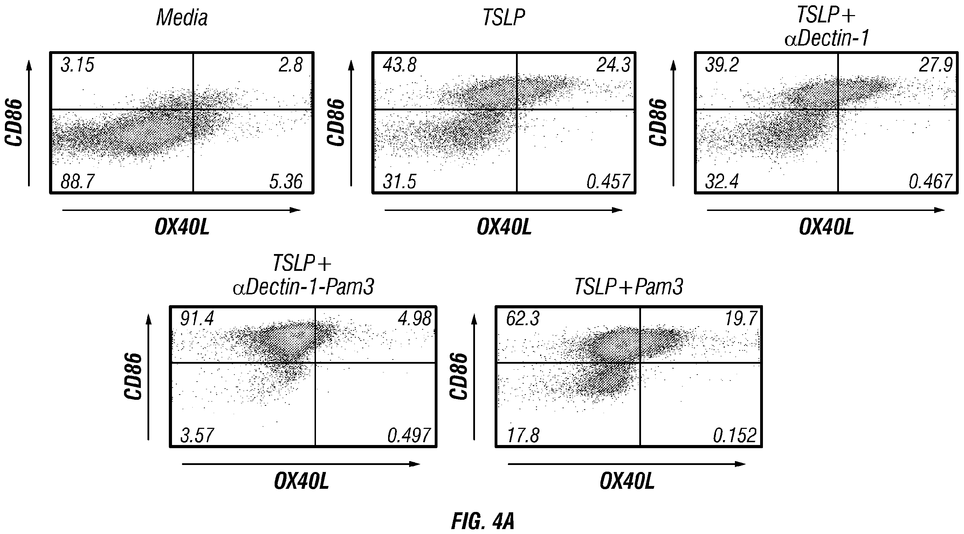

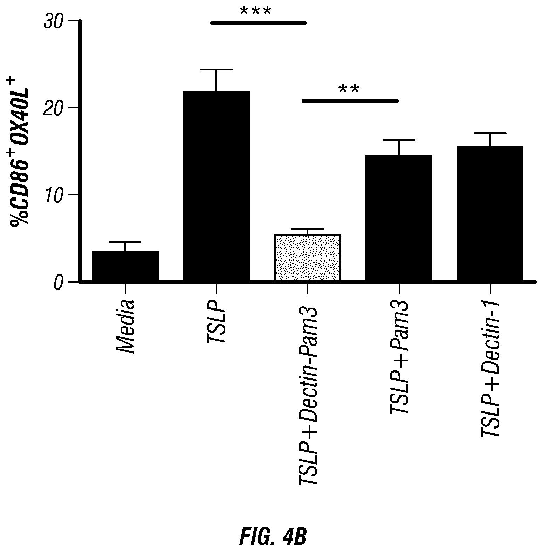

**Anti-Dectin-1-Pam3 conjugate can suppress TSLP-induced OX40L expression on mDCs**. Allergens and respiratory viruses induce epithelial cells to secrete TSLP that can upregulate OX40L expression on DCs. OX40L play a pivotal role in the DC-induced elicitation of allergic Th2 type inflammatory T cell responses. Thus, it was tested whether anti-Dectin-1-Pam3 could suppress the TSLP-induced OX40L expression on mDCs (**FIG. 4**). Compared to mDCs cultured overnight in the medium, mDCs cultured with TSLP expressed increased CD86 (activation marker) and OX40L. Anti-Dectin-1-Pam3 conjugate was able to promote TSLP-induced activation of mDCs (by looking at CD86 expression) whereas it decreased the TSLP-induced OX40L expression on mDCs. Either soluble form of anti-Dectin-1 antibody or Pam3 alone could not alter the TSLP-induced OX40L expression. Therefore, it is expected that anti-Dectin-1-Pam3 conjugate can effectively suppress Th2 type T cell responses elicited by TSLP-activated DCs.

**Anti-Dectin-1-Pam3 conjugate treatment results in the suppression of TSLP-activated mDC-induced Th2-type T cell responses**. It was next tested whether anti-Dectin-1-Pam3 conjugate can indeed suppress TSLP-activated mDC-induced Th2 type T cell responses. 5x10³ mDCs were cultured overnight with TSLP in the presence or absence of the same concentration (20 ug/ml) of anti-Dectin-1-Pam3 conjugate, combination of anti-Dectin-1 and Pam3, or Pam 3. 2x10⁵ purified naive CD4+ T cells were then added into the culture. After 7 days, T cells were stimulated for 6h with PMA/ionomycin in the presence of brefeldin A. Cells were then stained for intracellular expression of IL-13 (Th2 type cytokine) and IFNγ (Th2 type cytokine). As shown in **FIG. 5****,** anti-Dectin-1-Pam3 resulted in decreased IL-13+ CD4+ T cell responses (0.842%). Either combination of anti-Dectin-1 and Pam3 or Pam 3 alone did not decrease Th2 type T cell responses (3.3.24% or 3.47%, respectively). **FIG. 5** demonstrates that the conjugate reduces Th2-type T cell responses whereas coadministration of unconjugated anti-dectin-1 and Pam3 does not reduce Th2 type T cell responses.

### Example 2: To investigate whether anti-hDectin-1 Pam3 conjugate treatment down-regulates Th2-type T cell responses and IgE levels in vitro.

The effectiveness of anti-hDectin-1 mAb can be tested *in vitro* using PBMCs from patients. In this example, patients who are reactive to ragweed allergen in a prick test can be targeted. By targeting this group of patients, both allergen-specific and total T cell responses will be assessed. Allergen-specific and total Ig levels can also be measured. Assessments of total T cell responses and total Ig, especially IgE, levels may help to predict the effectiveness of anti-hDectin-1 mAb Pam3 conjugate in the down-regulation of other allergen-specific immune responses. In general, patients who are allergic to one allergen also show allergic reactions to different allergens as well in skin tests.

The following methods may be employed to test the in vitro effectiveness of the conjugate. Whole blood (60-80 ml per patient) from 20 allergic patients who show positive reaction to ragweed allergen in a prick test can be used. PBMCs and sera can then be prepared. Ragweed allergen-specific T cell responses can be assessed as described previously (CAMPBELL, J. D. ET AL. CLIN EXP ALLERGY 40, 1025-1035, (2010)). In brief, 200 µL of PBMC cultures at 5x10⁶ cells/ml can be incubated in 96-well plates with no antigen, defatted-ragweed allergen extract, or amb a 1 for 7 days in the presence of anti-hDectin-1 mAb conjugated to Pam3, control mAb, curdlan, or none. Both mouse and chimeric anti-hDectin-1 mAbs have similar capacity for binding to and for activating DCs (data not shown). The quantity and quality of antigen-specific T cells before and after *in vitro* culture can be assessed by ICS of CD154 and cytokines (IL-4, IL-5, IL-13, IL-10, IL-17, IL-21, IL-22, TNFα, and IFNγ) using multi-color flow cytometry (LSR II). Cytokines and chemokines secreted in culture supernatants after 48h stimulation of PBMCs before and after *in vitro* cultures will be measured by Luminex. PBMCs can be stimulated with ragweed allergen and phytohemagglutinin (PHA) for both ICS and Luminex. Total and ragweed antigen-specific Igs (IgM, IgG, IgA, and IgE) in sera will be assessed by ELISA. Sera from age- and sex-matched healthy can be used as controls. PBMC cultures in 96-well plates will be performed as described for T cell responses. On day 12, total and antigen-specific Ig levels in culture supernatants will be assessed by ELISASeveral comparisons can be made for T and B cell responses. These may include: 1) the levels of total and allergen-specific Th2-type responses between control and anti-hDectin-1 mAb Pam3 conjugates can be compared. Then, the ability of anti-hDectin-1 mAb Pam3 conjugate to down-regulate Th2-type responses can be compared with that of curdlan or curdlan plus Pam3 (unconjugated); 2) the quantity and quality of total and allergen-specific T cells before and after *in vitro* cultures can be compared by assessing the percentages and magnitudes of different types of T cells using ICS and Luminex data. Relative magnitudes of each type of T cells can be measured by assessing T cells expressing individual cytokines and combinations; 3) the levels of total and allergen-specific Igs, particularly IgE, in two groups, control and anti-hDectin-1 mAb Pam3 conjugates, can be compared. Then, Ig levels in the anti-hDectin-1-Pam3 treated group can be compared with those in the curdlan-treated group (or curdlan + Pam3-treated group); 4) comparative analyses for the associations between the levels of different types of T cell responses and the levels of Ig isotypes can be performed; and 5) the overall effectiveness of anti-hDectin-1-Pam3 in the presence and absence of antigens can be compared.

It is contemplated that anti-hDectin-1-Pam3 treatment will down-regulate total and

### Example 3: To investigate that anti-hDectin-1-Pam3 treatment down-regulates Th2-type immune responses and controls allergic atopy in NHP.

Anti-hDectin-1 mAb (15E2) cross-reacts with Dectin-1 in NHP, but not in mice. This allows one to test the effectiveness of anti-hDectin-1 mAb-Pam3 in the allergic atopy model of NHP. Intradermal route for the injection of mAb conjugates and HDMA mixtures may be used since DCs expressing Dectin-1 are mainly localized in the dermis of both human (Ni, et al., 2010) and monkey skin. As the first step of testing anti-hDectin-1 mAb Pam3 conjugates in an allergic disease model, additional i.v. injections of anti-hDectin-1 mAb Pam3 conjugates will be included. This will activate blood mDCs, resulting in further down-regulation of Th2-type immune responses. It is contemplated that anti-hDectin-1-Pam3 will be effective with or without co-injections of allergens. Anti-hDectin-1 mAb Pam3 conjugates and allergens may be injected simultaneously. This may help us to assess allergen-specific immune responses and treatment effect by comparing those after the injections of allergen alone.

The following methods may be employed to test the *in vivo* effectiveness of the conjugate. Young adult rhesus macaques (*Macaca mulatta,* female, age 3-5 years old) can be screened by skin test (commercial skin test kits for human usage). HDMA⁺ animals can be selected. Animals can be sensitized by s.c. injections of 25 µg house dust mite *(Dermatophagoides farinae*) allergen (HDMA: Greer Labs) in alum and i.d. injection of DtaP. All animals can be boosted four times by s.c. injections of 25 µg HDMA in alum (Schelegle, et al., 2001; Seshasayee, et al., 2007). Sensitization can be confirmed by skin test and by measuring serum Ig levels. Each animal can receive three doses of 25 µg HDMA in PBS at one week intervals in two sites at weeks 11-13. The same animals can be injected i.d. with three doses of 25 µg HDMA and 1 mg anti-hDectin-1 mAb Pam3 conjugate in PBS at two sites plus i.v. 1 mg of anti-hDectin-1 mAb Pam3 conjugate at weeks 15-17. Blood samples (7-10 ml per animal at each sampling date) can be collected in ACD tubes at weeks - 1, 0, 2, 4, 6, 8, 11, 13, 15, 17, 18, and 20. PBMCs and sera can be prepared. On weeks 14 and 18, animals can receive i.d. injections of 12.5 µg HDMA per site (4 sites per animals) and skin reaction can be measured. Skin biopsies can also be taken after 48-72h, and 2 biopsies per animals can be frozen in OCT medium. The other two biopsies can be used for measuring IgE level after washing small pieces with 500 µl PBS. Serum cytokines (IL-4, IL-5, IL-10, IL-13, IL-17, IL-21, IL-22, TNFα, and IFNγ) can be assessed by Luminex. Total and HDMA-specific Ig levels can be assessed by ELISA by previously known methods (Schelegle, et al., 2001; Seshasayee, et al., 2007). Pooled human HDMA IgE-positive sera (RAST tested high level) can be used as positive controls. Negative controls may consist of PBS and serum from HDMA skin-test-negative animals. PBMCs and T cells enriched with commercial enrichment kits will be incubated overnight in the presence or absence of 50 µg SEB. Cytokines in the culture supernatants can be measured by Luminex. T cells can be stained for intracellular IL-4, IL-5, IL-10, IL-13, IL-17, IL-21, IL-22, TNFα, and IFNγ. Sections of frozen skin biopsies can be stained for DCs (Park, et al., 2008; Gros, et al., 2009), eosinophils, neutrophils, basophils, and memory/naïve T cells (Park, et al., 2008; Gros, et al., 2009; Simon, et al., 2011; Spergel, et al., 2005; Langeveld-Wildschut, et al., 1996; Hogan, et al., 2008; Menzies-Gow, et al., 2002; Gaga, et al., 2008). The following may be assessed: 1) serum IgE levels before and after sensitization, after 3 doses of HDMA (control group), and after three doses of HDMA plus anti-hDectin-1 mAb Pam3 conjugate (experimental group) can be compared; 2) serum cytokine levels can be assessed and compared at each time point; 3) the frequency of T cells expressing single cytokines and combinations, particularly IL-17 and Th2-type cytokines, can be compared at each time point; 4) the amounts of cytokines secreted by total PBMCs and enriched T cell populations can be compared at each time point; 5) the frequency of DCs, eosinophils, neutrophils, basophils, and lymphocytes infiltrated into the skin can be compared; 6) skin reaction and IgE after HDMA injections on weeks 14 and 18 can be assessed and compared.

It is contemplated that anti-hDectin-1-Pam3 treatment is expected to result in decreased Th2-type T cell responses, IgE levels, lymphocyte infiltration, and skin reaction.

### Example 4: TLR Conjugate synthesis.

This example demonstrates the conjugation of a TLR2, Pam3-CSK4 to an antibody.

### Peptide Coupling between H₂NSK₄CK-biotin resin and PAM₃Cys-OH (Scheme 1)

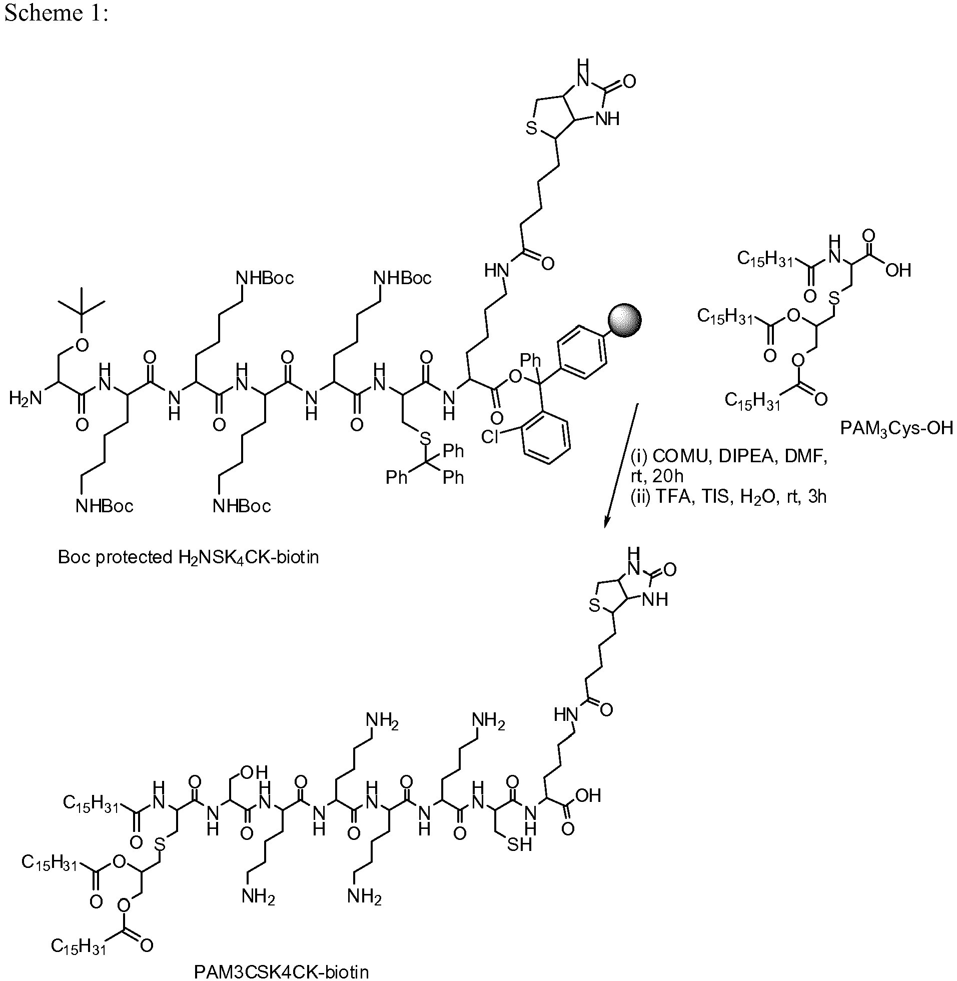

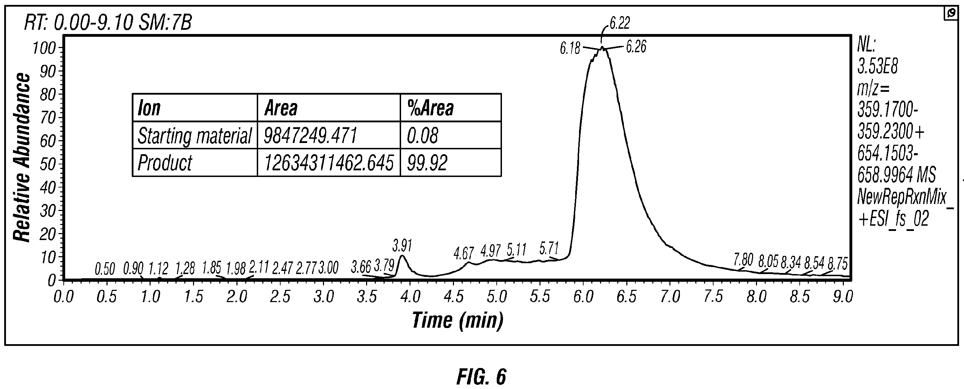

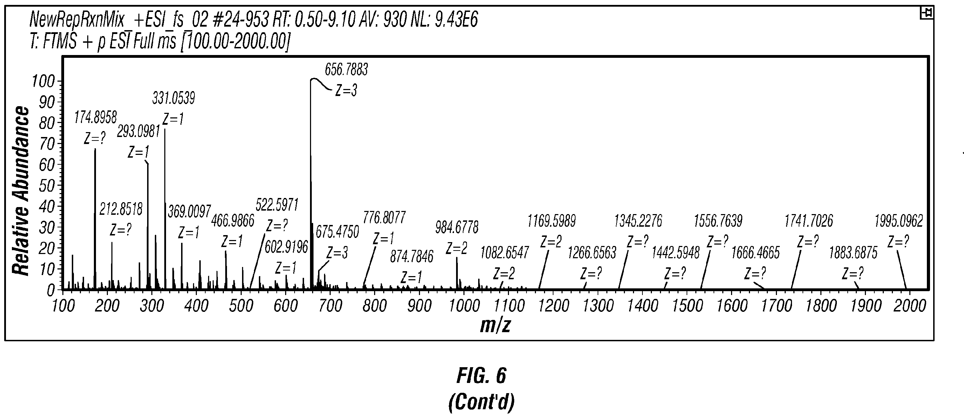

PAM₃Cys-OH (60 mg, 0.07 mmol) was dissolved with 0.6 ml dichloromethane in a clean reaction vial. N,N-diisopropylethylamine (0.02 ml, 0.11 mmol), COMU (28 mg, 0.07 mmol) and DMF (0.2 ml) were added. After thoroughly shaking the mixture, the reaction vial was allowed to stand for 20 minutes. H₂NSK₄CK-biotin resin (14.1 mg) was added and reaction was allowed for 20 hours with occasional swirling. The resin was filtered (using DMF to rinse onto a fritted glass funnel) and transferred to another vial. The cleaving cocktail (561 µL TFA, 31 µL water, 18 µL triisopropylsilane) was added to the resin in this vial. After swirling the vial occasionally for 3 hours, the resin was filtered using a glass-wool plugged Pasteur pipette and the filtrate was evaporated to give 7.3 mg of product (PAM₃CSK₄CK-biotin). The chromatogram and mass spectra of the product is shown in FIG. 6.

### Synthesis of PAM3-biotin-DBCO (Scheme 2)

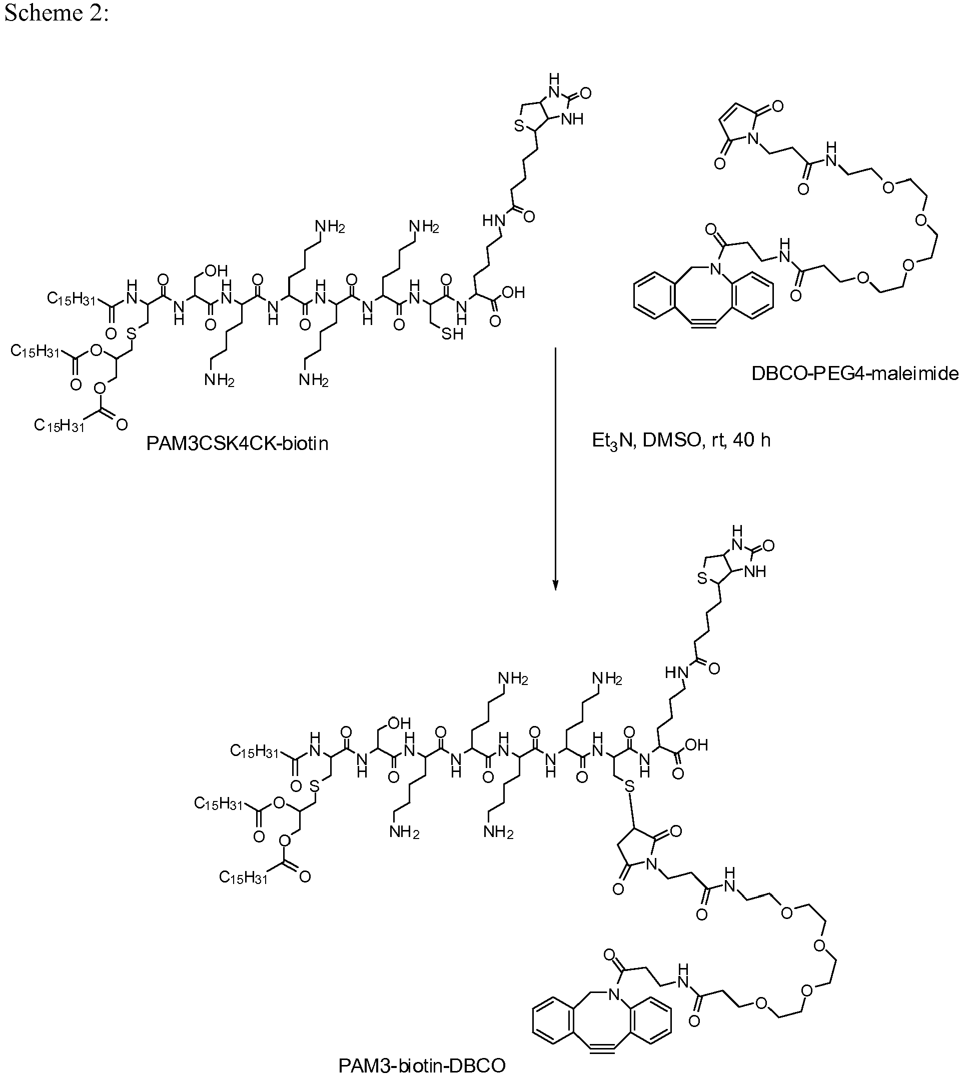

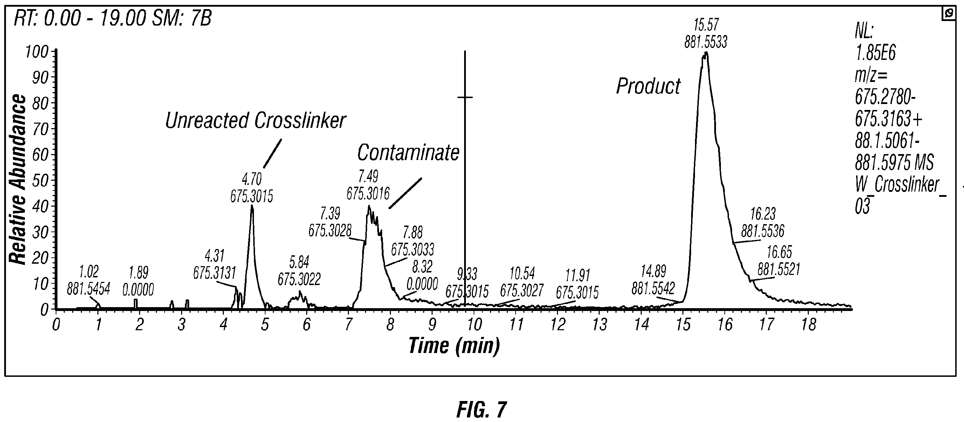

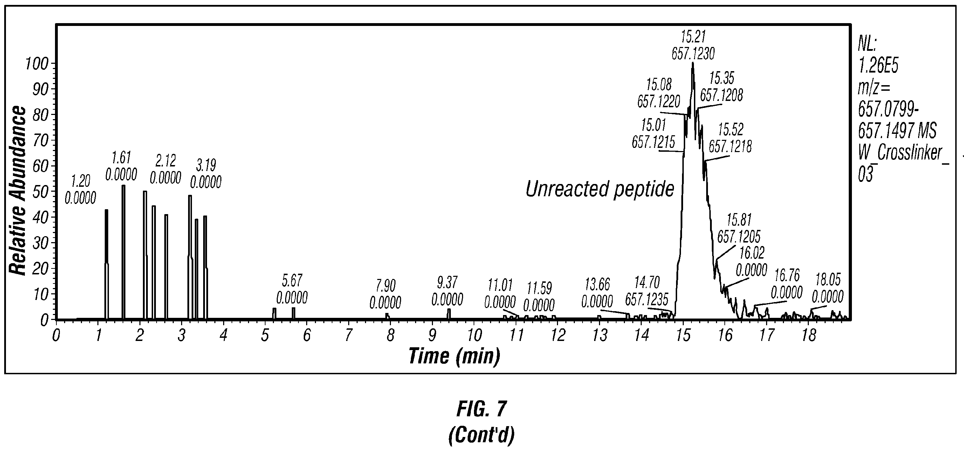

DBCO-PEG4-maleimide (2 mg, 3 µmol) was dissolved in 0.4 ml DMSO. PAM3CSK4CK-biotin (as synthesized above in Scheme 1; 7.3 mg, 3 µmol) and triethylamine (7.3 µL, 52 µmol) were added. The mixture was stirred for 40 hours at room temperature. The extracted chromatogram of the product is shown in FIG. 7. The unreacted peptide was observed at 656.4527. And is predicted to have a theoretical mass of 656.4547. The unreacted crosslinker was observed at 675.3020 and is predicted to have a theoretical mass of 675.3032. The product was observed at 881.2185 and is predicted to have a theoretical mass of 881.2194. (Note that the peptide and product were triply charged, and the proton mass of 1.008 was used.). The extracted chromatogram, that shows a small amount of unreacted peptide and crosslinker, as well as product. (The unreacted peptide co-elutes with the product, so it is shown on a seperate chromatogram). In regards to peak area- unreacted peptide is at 8.6%, unreacted crosslinker is at 4.49%, and the product is 86.91%.

### Conjugation of PAM3-Biotin-DBCO Molecule to Antibody (Scheme 3)

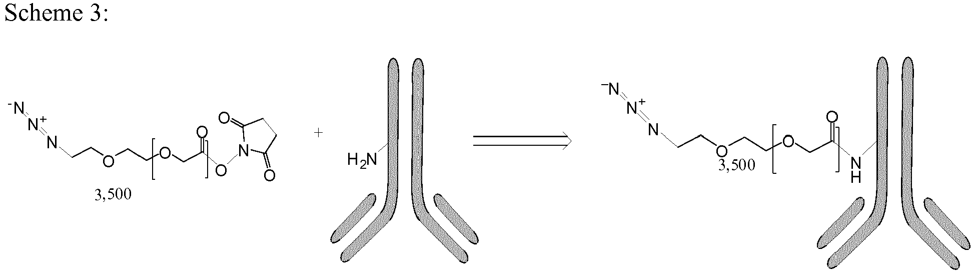

### Conjugation of PAM3-Biotin-DBCO Molecule to Antibody (Scheme 4)

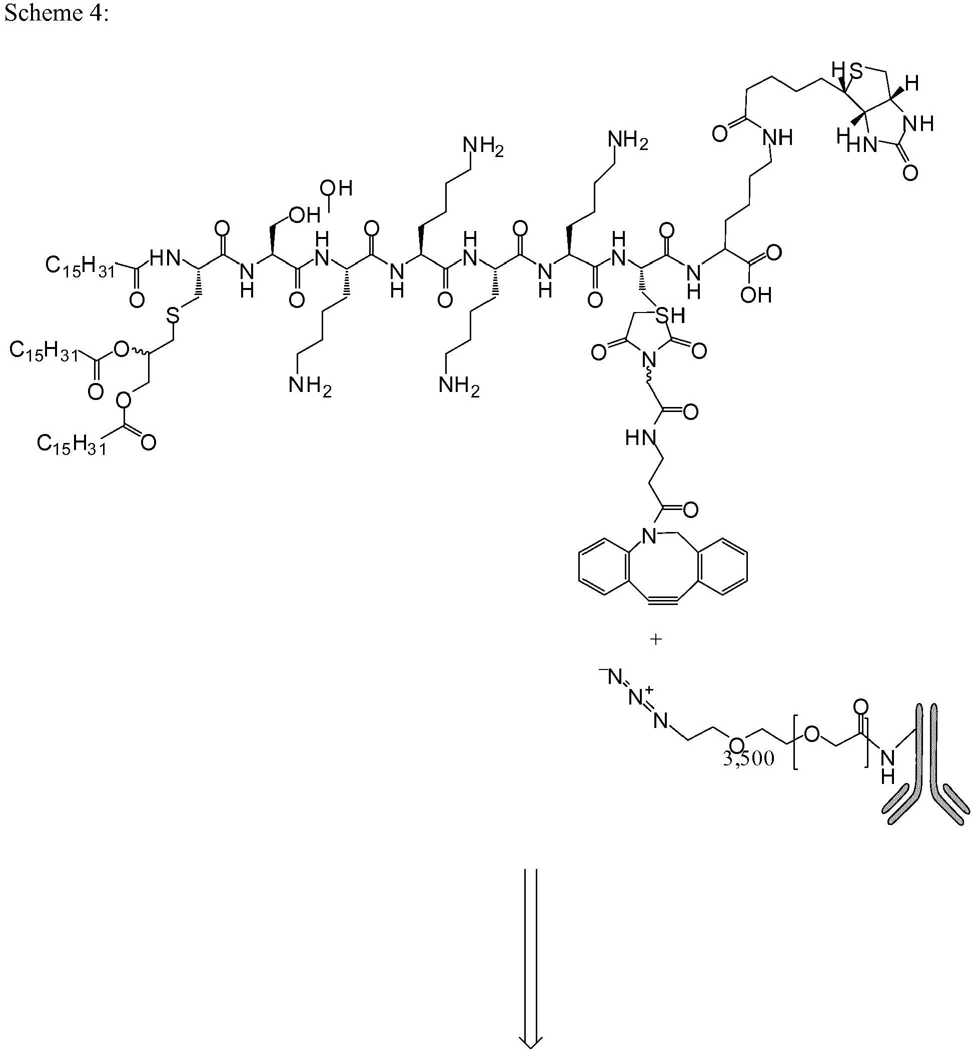

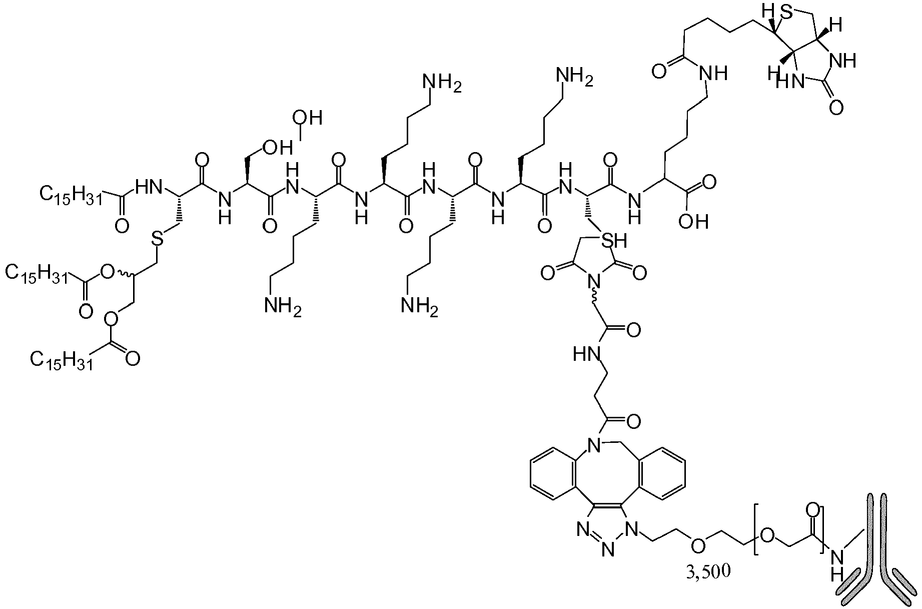

The PAM3-Biotin-DBCO was then conjugated to the antibody. 93.7 µL of 1mM NHS-PEG3500-Azide (66.7 nmols) in DMSO and 288µL of 5.2 mg/ml IgG (6.67 nmols) were added to 862 µL PBS (Scheme 3). The solution was incubated for 2 hours on ice protected from light. The reaction was quenched with 2 µL of 2M Tris buffer and incubated on ice for 15 min. The reaction mixture was dialyzed on a 7,000 MWCO slide-a-lyzer in PBS to remove excess non-reacted NHS ester. 28.1 µL of 1 mM PAM3-Biotin-DBCO working solution (20 nmols) was added to the dialyzed IgG-PEG3500-Azide product (Scheme 4). This mixture was incubated for 24 hours at 4°C. The reaction mixture is dialyzed on a 7,000 MWCO slide-a-lyzer in PBS to remove un-reacted PAM3-Biotin-DBCO.

### Example 5: Pam3CSK4 and NHS-PEG-Azide Conjugation and Dectin

The following method can be used in the conjugation of Pam3CSK4 to Dectin antibody.

187.5 µL of 1 mM NHS-Phosphine (187.5 nmols) in DMSO can be mixed with 552.5 µL of 5.22 mg/ml anti-hDectin-1 Antibody(18.8 nmols) in 1238 µL DPBS. The mixture can then be incubated for 2 hours on ice protected from light. The reaction can be quenched with 2 uL of 2M Tris buffer and can then be incubated on ice for 15 min. A 7,000 MWCO slide-a-lyzer in DPBS may be used to remove excess non-reacted NHS ester. 85 µL of 664 µM PAM3CSK4 (56.4 nmols) in endotoxin free water and 85µE of 664 µM NHS-PEG-Azide (56.4 nmols) in DPBS can then be added into 1,307 µL of DPBS at a pH above 7. This reaction mixture can then be incubated on ice for 2 hours. Next, the phosphinylated anti-hDectin-1 Antibody can be added, and the mixture may then be incubated for twenty-four hours on ice. The 7,000 MWCO slide-a-lyzer can then be used to remove un-reacted Pam3-PEG-Azide molecules.

### Example 6: Preclinical Assessment of the Effectiveness of αDectin-1-Pam3 Conjugate in Controlling TH2 Responses

Dectin-1 is a pattern recognition receptor, which contributes to both innate and adaptive immunity against certain fungal and bacterial infections. Previously, Applicants had shown that signals via Dectin-1 and TLR2 synergize to activate DCs, resulting in decreased TH2 responses. In this example, Applicants have made α-hDectin-1-Pam3CSK4 (Pam3) conjugate and tested its effectiveness in the suppression of TH2 responses in human in vitro and non-human primates (NHP) in vivo.

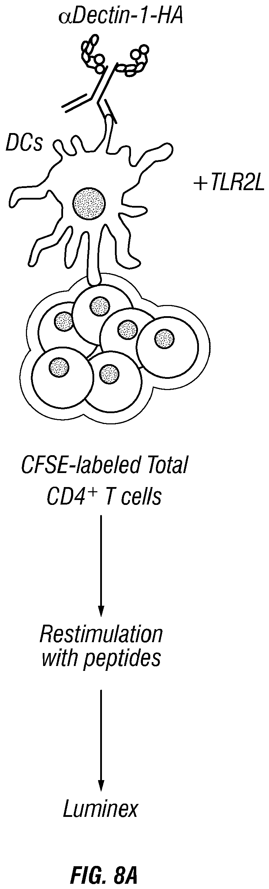

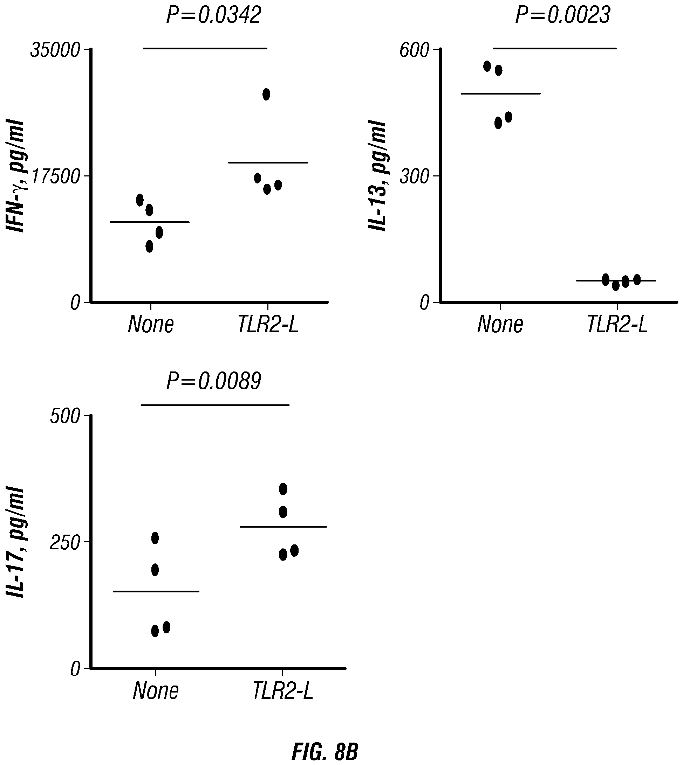

The addition of TLR2-L to Dectin-1 activation leads to decreased HA-1 specific Th2-type CD4+ T Cell responses. CFSE-labeled CD4+ T cells were co-cultured for 7 days with DCs loaded with either αDectin-1-HA alone or αDectin-1-HA plus TLR2-L. T cells were re-stimulated with HA1 peptides and Cytokine levels were analyzed by Luminex (FIG. 8A). As shown in FIG. 8B, the addition of TLR2-L to Dectin-1 activation leads to increased IFN-γ, decreased IL-13, and increased IL-17 production (FIG. 8B).

As shown in FIG. 1, a linker is attached to pam3CSK4 to help increase solubility and to prevent crosslinking of multiple pam3 molecules. A phosphine group is added to the αDectin-1, which can then react with the free azide on the Pam3CSK3, thus creating a conjugate between the two compounds.

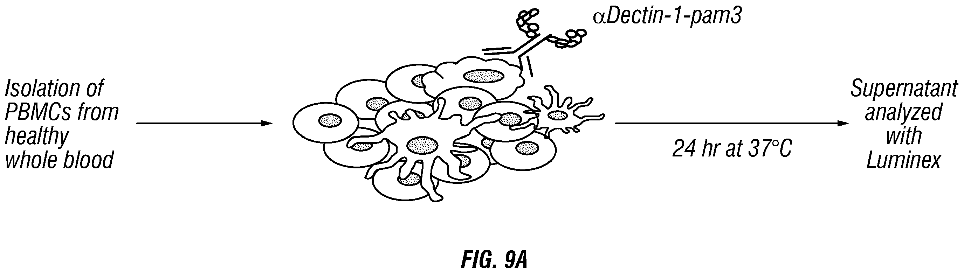

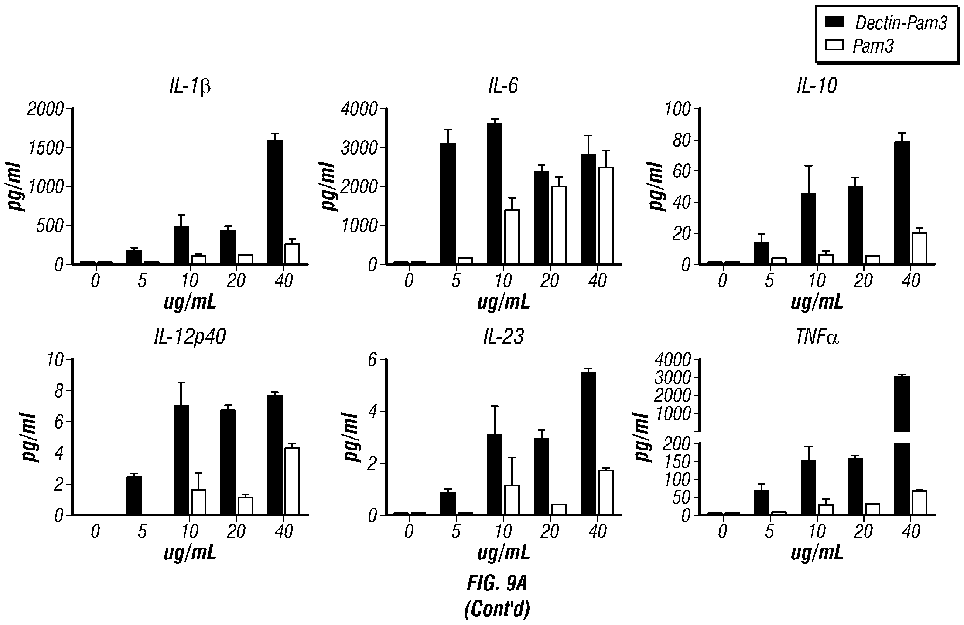

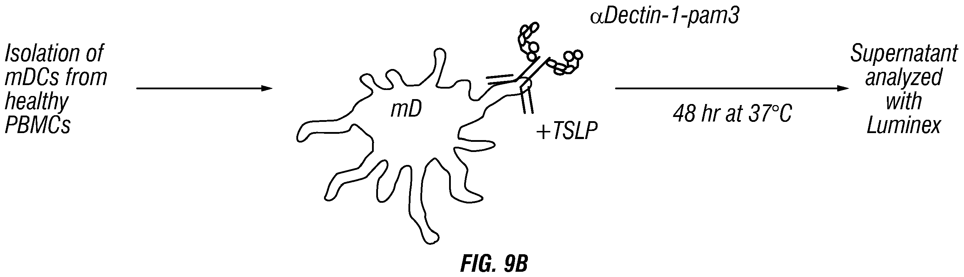

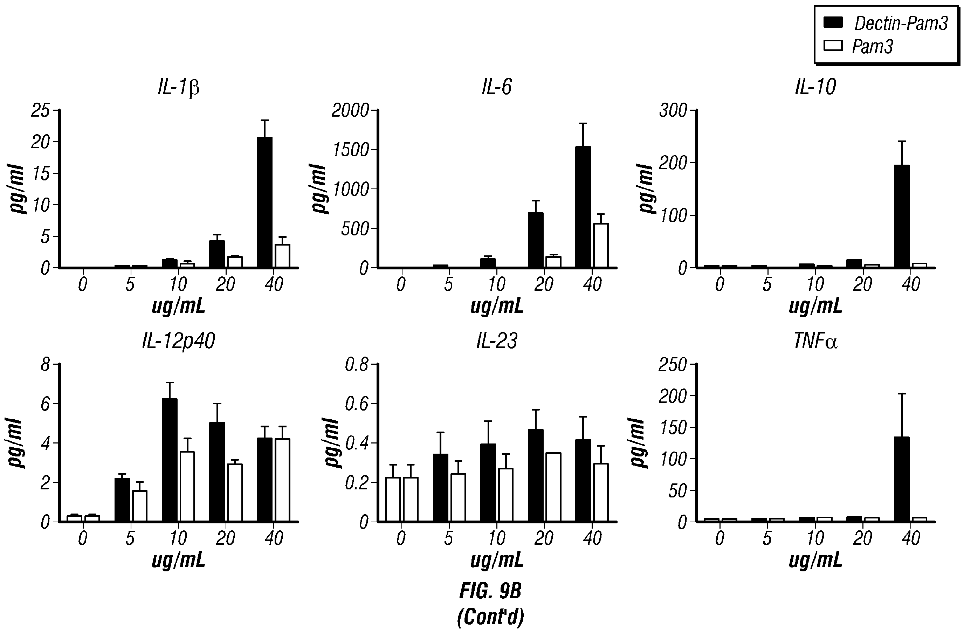

Binding capacity of antibody and pam3 conjugates were tested in PBMCs, and the TLR2 signaling activity of TLR2 reporter cells with titrated amounts of either αDectin-1, pam3 or αDectin-1-pam3 were tested. As shown in FIG. 2, αDectin-1-pam3 has no loss of binding (FIG. 2A) and relatively unchanged TLR2 activity (FIG. 2B). Next, PBMCs (FIG. 9A) and mDCs (FIG. 9B) were cultured for 24 to 48 hours, then supernatants were harvested for Luminex analysis. αDectin-1-Pam3 activates cells in a titration-dependent manner (FIG. 9A-B).

Next, mDCs were first purified from a buffy coat then cultured with 20 ng/mL TSLP and either 100 ng/mL pam3, 10 µg/mL of anti-dectin-1 or 10 µg/mL of αDectin-1-pam3. Cells were harvested and stained after 48 hours. As shown in FIG. 4A-B, αDectin-1-pam3 conjugate can decrease TSLP-induced OX40L expression on blood mDCs. FIG. 4A shows mDC staining, and FIG. 4B shows the compiled results.

Next, the Th2-type T cells were tested. mDCs were first primed with 40 ng/mL TSLP and either αdectin-1 or αDectin-1-pam3 at 20 ug/mL. After 24 hrs, naive CD4+ T cells are added to the mDCs and cultured for an additional 6 days. Intracellular cytokine levels were analyzed by intracellular staining in cells stimulated with PMA/Ionomycin for 6 hours and with brefeldinA for 4 hrs (FIG. 10A). Cell supernatant cytokine levels were measured by stimulating the cells with αCD3/CD28 beads for 48 hrs (FIG. 10B). As shown in FIG. 10, αDectin-1-pam3 conjugate can decrease TSLP-mDC induced TH2-type CD4+ T cell responses while promoting TH1- and TH17-type CD4+ T cells responses.

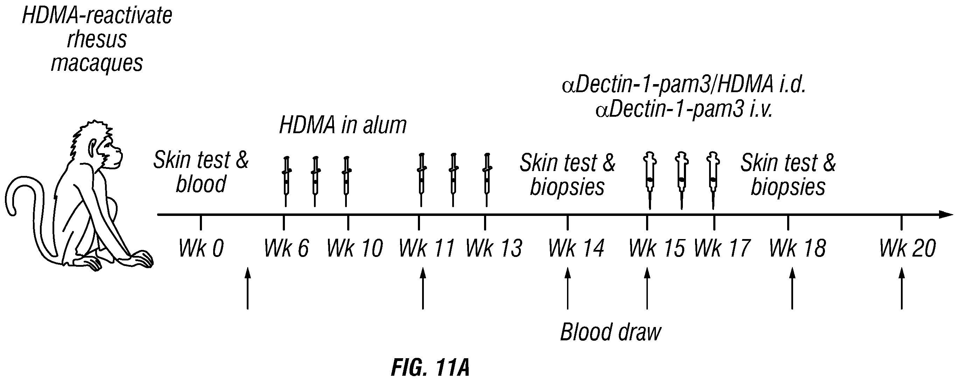

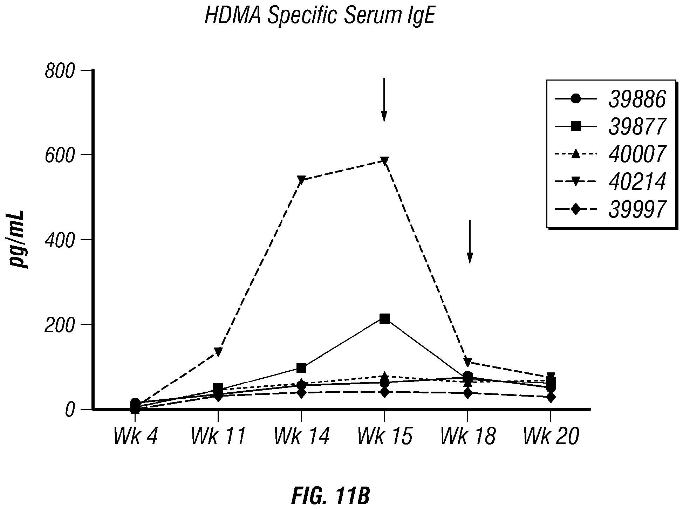

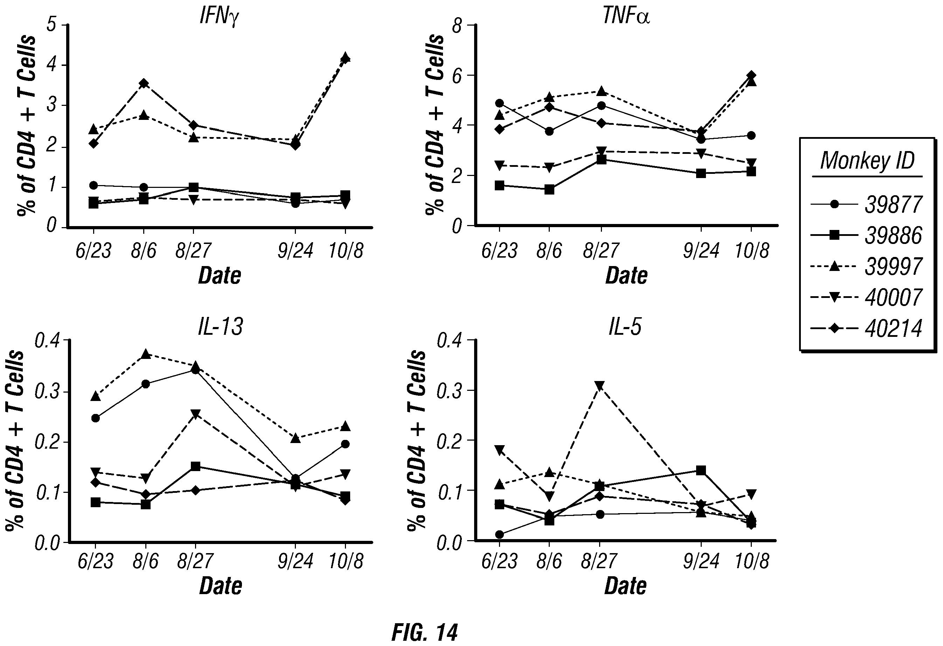

Last, HDMA-specific serum IgE was tested in HDMA-reactive rhesus macaques. NHP model for atropy was generated by sensitizing the animals to HDMA (FIG. 11A). HDMA-specific serum IgE was then measured. αDectin-1 Pam3 treatment decreases HDMA-specific serum IgE *in vivo* (FIG. 11B). FIG. 14 shows the intracellular cytokine signaling from the serum of these animals taken during the course of this experiment.

These results show that concomitant activation of DCs through Dectin-1 and TLR2 can significantly decrease TH2 responses while slightly enhancing TH1- and TH17 responses in human *in vitro.* Furthermore, αDectin-1-pam3 can decrease HDMA-specific serum IgE responses in non-human primate in vivo. The αDectin-1-pam3 conjugate could be a novel therapeutic candidate for TH2-driven inflammatory diseases.

### REFERENCES

Allakhverdi, et al., J Exp Med 204, 253-258, (2007).
Ballow, J Allergy Clin Immunol 118, 1209-1215; quiz 1216-1207, (2006).
Banchereau & Steinman, Nature 392, 245-252, (1998).
Barnes, J Clin Invest 118, 3546-3556, (2008).
Bellinghausen, et al., Int Arch Allergy Immunol 126, 97-101, (2001).
Bieneman, et al., J Allergy Clin Immunol 115, 295-301, (2005).
Bunyavanich, et al., Clin Mol Allergy 9, 1, (2011).
Campbell, et al., Clin Exp Allergy 40, 1025-1035, (2010).
Corren, et al., Am J Respir Crit Care Med 181, 788-796, (2010).
Cox & Wallace, Immunol Allergy Clin North Am 31, 561-599, (2011).
Ferwerda, et al., N Engl J Med 361, 1760-1767, (2009).
Flood-Page, et al., Am J Respir Crit Care Med 176, 1062-1071, (2007).
Fukahori, et al., Clin Exp Allergy 40, 1507-1515, (2010).
Gaga, et al., Allergy 63, 703-711, (2008).
Gros, et al., J Allergy Clin Immunol 124, 753-760 e751, (2009).
Haldar, et al., N Engl J Med 360, 973-984, (2009).
Herbert, et al., Am J Pathol 177, 1657-1664, (2010).
Hessel, et al., J Exp Med 202, 1563-1573, (2005).
Hogan, et al., Clin Exp Allergy 38, 709-750, (2008).
Horvat, et al., J Immunol 184, 4159-4169, (2010).
Ito, et al., Proc Natl Acad Sci U S A 103, 13138-13143, (2006).
Ito, et al., J Exp Med 202, 1213-1223, (2005).
Jutel & Akdis, Allergy 66, 725-732, (2011).
Kips, et al., Am J Respir Crit Care Med 167, 1655-1659, (2003).
Krawiec & Wenzel, Expert Opin Pharmacother 2, 47-65, (2001).
Laan, et al., J Immunol 162, 2347-2352, (1999).
Lajoie, et al., Nat Immunol 11, 928-935, (2010).
Langeveld-Wildschut, et al., J Allergy Clin Immunol 98, 1019-1027, (1996).
Larsson, et al., Int Arch Allergy Immunol 149, 1-15, (2009).
LeibundGut-Landmann, et al., Nat Immunol 8, 630-638, (2007).
Levine & Wenzel, Ann Intern Med 152, 232-237, (2010).
Lindner, et al., J Leukoc Biol 86, 389-399, (2009).
McCurdy, et al., J Immunol 170, 1625-1629, (2003).
McKinley, et al., J Immunol 181, 4089-4097, (2008).
Menzies-Gow, et al., J Immunol 169, 2712-2718, (2002).
Menzies-Gow, et al., Clin Exp Allergy 37, 1023-1032, (2007).
Meyer-Wentrup, et al., Blood 111, 4245-4253, (2008).
Mionnet, et al., Nat Med 16, 1305-1312, (2010).
Mohapatra, et al., Curr Opin Pharmacol 10, 276-288, (2010).
Morita, et al., Immunity 34, 108-121, (2011).
Murdoch & Lloyd, Am J Respir Crit Care Med 182, 464-476, (2010).
Muzzarelli, Mar Drugs 8, 292-312, (2010).
Nakae, et al., Immunity 17, 375-387, (2002).
Neurath, et al., Nat Med 8, 567-573, (2002).
Ni, et al., J Immunol 185, 3504-3513, (2010).
Olynych, et al., J Allergy Clin Immunol 118, 837-843, (2006).
Park, et al., Exp Dermatol 17, 24-29, (2008).
Reddy, et al., J Immunol 164, 1925-1933, (2000).
Ribbing, et al., Allergy 66, 110-119, (2011).
Rochman, et al., J Immunol 178, 6720-6724, (2007).
Schelegle, et al., Am J 158, 333-341, (2001).
Schnyder-Candrian, et al. J Exp Med 203, 2715-2725, (2006).
Seshasayee, et al., J Clin Invest 117, 3868-3878, (2007).
Simon, et al., J Allergy Clin Immunol 127, 194-199, (2011).
Singh, et al., BMC Pulm Med 10, 3, (2010).
Sorgi, et al., J Immunol 182, 4025-4035, (2009).
Soyer, et al., Immunol Allergy Clin North Am 31, 175-190, vii-viii, (2011).
Spergel, et al., Ann Allergy Asthma Immunol 95, 336-343, (2005).
Steinman, et al., Annu Rev Immunol 21, 685-711, (2003).
Taylor, et al., Nat Immunol 8, 31-38, (2007).
Taylor, et al., J Immunol 169, 3876-3882, (2002).
Tomkinson, et al., Allergy 65, 69-77, (2010).
Valera, et al., J Immunol 180, 5727-5736, (2008).
Wang, et al., J Exp Med 207, 2479-2491, (2010).
Wenzel, et al., Lancet 370, 1422-1431, (2007).
Willment, et al., Eur J Immunol 35, 1539-1547, (2005).
Wilson, et al., Am JRespir Crit Care Med 180, 720-730, (2009).
Yang, et al., J Exp Med 205, 1063-1075, (2008).
Ying, et al., J Immunol 174, 8183-8190, (2005).
Yoon, et al., J Immunol 181, 2907-2915, (2008).

## Claims

1. A therapeutically effective amount of an anti-Dectin-1 antibody or antigen binding fragment thereof operatively linked to Pam3CSK4 for use in a method for preventing or treating allergic and inflammatory disorders in a subject in need thereof comprising administering to the subject the therapeutically effective amount of the anti-Dectin-1 antibody or antigen binding fragment thereof operatively linked to Pam3CSK4.

2. The anti-Dectin-1 antibody or antigen binding fragment thereof operatively linked to Pam3CSK4 for use according to claim 1, wherein:
(i) Pam3CSK4 is conjugated to the anti-Dectin-1 antibody or antigen binding fragment thereof; and/or
(ii) Pam3CSK4 is chemically conjugated to the anti-Dectin-1 antibody or antigen binding fragment thereof; and/or
(iii) the antibody or antigen binding fragment specifically binds to dectin-1 and activates dectin-1; and/or
(iv) the anti-Dectin-1 antibody or antigen binding fragment thereof is a human antibody, humanized antibody, recombinant antibody, chimeric antibody, an antibody derivative, a veneered antibody, a diabody, a monoclonal antibody, or a polyclonal antibody.

3. The anti-Dectin-1 antibody or antigen binding fragment thereof operatively linked to Pam3CSK4 for use according to claim 1 or claim 2, wherein:
(i) the antibody comprises a variable region comprising an amino acid sequence selected from the sequences of SEQ ID NOs:2, 4, 6, 8, 10, and 12; or
(ii) the antibody comprises a CDR having an amino acid sequence corresponding to any one of SEQ ID NOs:13-30; or
(iii) the antibody comprises a heavy chain comprising CDRs having an amino acid sequence corresponding to SEQ ID NO: 13-15 and a light chain having an amino acid sequence corresponding to SEQ ID NO:16-18; or
(iv) the antibody comprises a heavy chain comprising CDRs having an amino acid sequence corresponding to SEQ ID NO: 19-21 and a light chain having an amino acid sequence corresponding to SEQ ID NO:22-24; or
(v) the antibody comprises a heavy chain comprising CDRs having an amino acid sequence corresponding to SEQ ID NO:25-27 and a light chain having an amino acid sequence corresponding to SEQ ID NO:28-30 or
(vi) the antibody comprises a heavy or light chain with an amino acid sequence selected from the sequences of SEQ ID NOs: 1, 3, 5, 7, 9, and 11 or
(vii) the antibody or antigen binding fragment further comprises a modification and the modification is:
a conservative amino acid mutation within the VH and/or VL CDR 1, CDR 2 and/or CDR 3 regions; or
a conservative amino acid mutation in the Fc hinge region; or
pegylation; or
conjugation to a serum protein; or
conjugation to human serum albumin; or
conjugation to a detectable label; or
conjugation to a diagnostic agent; or
conjugation to an enzyme; or
conjugation to a fluorescent, luminescent, or bioluminescent material; or
conjugation to a radioactive material; or
conjugation to a therapeutic agent.

4. The anti-Dectin-1 antibody or antigen binding fragment thereof operatively linked to Pam3CSK4 for use according to any preceding claim, wherein:
(i) the anti-Dectin-1 antibody or antigen binding fragment thereof comprises a γ4 constant region; and/or
(ii) the anti-Dectin-1 antibody or antigen binding fragment thereof comprises a γ4 constant region and the γ4 constant region comprises a substitution of glutamic acid for leucine at residue 235; and/or
(iii) the anti-Dectin-1 antibody or antigen binding fragment thereof comprises a γ4 constant region and the γ4 constant region comprises a substitution of proline for serine at residue 228 in the hinge region; and/or
(iv) the subject is a human subject; and/or
(v) the treatment is for an allergic disorder; and/or
(vi) the treatment is for a TH2-mediated allergic disorder; and/or
(vii) the treatment is for a TH2 mediated inflammatory disorder; and/or
(viii) the treatment is for a TH2 mediated inflammatory disorder which is selected from such as asthma, chronic obstructive pulmonary disease, interstitial lung disease, chronic obstructive lung disease, chronic bronchitis, eosinophilic bronchitis, eosinophilic pneumonia, pneumonia, inflammatory bowel disease, atopic dermatitis, atopy, allergy, allergic rhinitis, idiopathic pulmonary fibrosis, scleroderma, emphysema, breast cancer, and ulcerative colitis; and/or
(ix) the treatment is for a TH2 mediated inflammatory disorder which is breast cancer; and/or
(x) the treatment is for a TH2 mediated inflammatory disorder which is ulcerative colitis; and/or
(xi) the subject is suffering from or is at risk of suffering from type 2 diabetes; and/or
(xii) the subject has an allergic disorder and the antibody or antigen binding fragment thereof operatively linked to Pam3CSK4 is administered prior to onset of an allergic reaction; or the subject has an allergic disorder and the antibody or antigen binding fragment thereof is administered after onset of an allergic reaction; and/or
(xiii) the anti-Dectin-1 antibody or antigen binding fragment thereof operatively linked to Pam3CSK4 is administered in an amount effective for the increase of one or more of Th1, Th17, and Treg cells in the subject; and/or
(xiv) the anti-Dectin-1 antibody or antigen binding fragment thereof operatively linked to Pam3CSK4 is administered by intradermal injection or by intravenous injection; and/or
(xv) the antibody or antigen binding fragment further comprises a modification; and/or
(xvi) the anti-Dectin-1 antibody or antigen binding fragment thereof operatively linked to Pam3CSK4 is administered in a pharmaceutical composition; and/or
(xvii) the anti-Dectin-1 antibody or antigen binding fragment thereof operatively linked to Pam3CSK4 is administered in a pharmaceutical composition and the pharmaceutical composition does not contain an antigen or allergen; and/or
(xviii) the antibody is administered in a pharmaceutical composition and the pharmaceutical composition consists essentially of an anti-dectin-1 antibody or antigen binding fragment thereof operatively linked to Pam3CSK4; and/or
(xix) the antibody or antigen binding fragment thereof is not conjugated to an antigen; and/or
(xx) the antibody is not conjugated to a dockerin or cohesin molecule.

5. A pharmaceutical composition comprising a therapeutically effective amount of an anti-Dectin-1 antibody or antigen binding fragment thereof operatively linked to Pam3CSK4.

6. The pharmaceutical composition of claim 5, wherein:
(i) Pam3CSK4 is conjugated to the anti-Dectin-1 antibody or antigen binding fragment thereof; and/or
(ii) the antibody or antigen binding fragment specifically binds to dectin-1 and activates dectin-1; and/or
(iii) the antibody or antigen binding fragment thereof binds to human dectin-1; and/or
(iv) the anti-Dectin-1 antibody or antigen binding fragment thereof is a human antibody, humanized antibody, recombinant antibody, chimeric antibody, an antibody derivative, a veneered antibody, a diabody, a monoclonal antibody, or a polyclonal antibody; and/or
(v) the anti-Dectin-1 antibody or antigen binding fragment thereof comprises a γ4 constant region; and/or
(vi) the anti-Dectin-1 antibody or antigen binding fragment thereof comprises a γ4 constant region and the γ4 constant region comprises a substitution of glutamic acid for leucine at residue 235; and/or
(vii) the anti-Dectin-1 antibody or antigen binding fragment thereof comprises a γ4 constant region and the γ4 constant region comprises a substitution of proline for serine at residue 228 in the hinge region; and/or
(viii) the anti-Dectin-1 antibody or antigen binding fragment thereof operatively linked to Pam3CSK4 is in an amount effective for the increase of one or more of Th1, Th17, and Treg cells in the subject; and/or
(ix) the composition is formulated for intradermal injection; and/or
(x) the composition is formulated for intravenous injection; and/or
(xi) the antibody or antigen binding fragment further comprises a modification; and/or
(xii) the pharmaceutical composition does not contain an antigen or allergen; and/or
(xiii) the pharmaceutical composition consists essentially of an anti-dectin-1 antibody or antigen binding fragment thereof operatively linked to Pam3CSK4; and/or
(xiv) the antibody or antigen binding fragment thereof is not conjugated to an antigen; and/or
(xv) the antibody is not conjugated to a dockerin or cohesin molecule.

7. The pharmaceutical composition of claim 6(i), wherein Pam3CSK4 is chemically conjugated to the anti-Dectin-1 antibody or antigen binding fragment thereof.

8. The pharmaceutical composition of any one of claims 5-7, wherein the antibody comprises a variable region comprising an amino acid sequence selected from the sequences of SEQ ID NOs: 2, 4, 6, 8, 10, and 12.

9. The pharmaceutical composition of any one of claims 5-7, wherein the antibody comprises a CDR having an amino acid sequence corresponding to any one of SEQ ID NOs: 13-30.

10. The pharmaceutical composition of any one of claims 5-9, wherein:
(i) the antibody comprises a heavy chain comprising CDRs having an amino acid sequence corresponding to SEQ ID NO: 13-15 and a light chain having an amino acid sequence corresponding to SEQ ID NO: 16-18; or
(ii) the antibody comprises a heavy chain comprising CDRs having an amino acid sequence corresponding to SEQ ID NO: 19-21 and a light chain having an amino acid sequence corresponding to SEQ ID NO: 22-24; or
(iii) the antibody comprises a heavy chain comprising CDRs having an amino acid sequence corresponding to SEQ ID NO: 25-27 and a light chain having an amino acid sequence corresponding to SEQ ID NO: 28-30.

11. The pharmaceutical composition of any one of claims 5-9, wherein the antibody comprises a heavy or light chain with an amino acid sequence selected from the sequences of SEQ ID NOs: 1, 3, 5, 7, 9, and 11.

12. The pharmaceutical composition of claim 6(xi), wherein the modification is:
a conservative amino acid mutation within the VH and/or VL CDR 1, CDR 2 and/or CDR 3 regions; or
a conservative amino acid mutation in the Fc hinge region; or
pegylation; or
conjugation to a serum protein; or
conjugation to human serum albumin; or
conjugation to a detectable label; or
conjugation to a diagnostic agent; or
conjugation to an enzyme; or
conjugation to a fluorescent, luminescent, or bioluminescent material; or
conjugation to a radioactive material; or
conjugation to a therapeutic agent.

13. A pharmaceutical composition according to any one of claims 6-12 for use in a method for:
(i) decreasing Th2-type cell responses in a subject in need thereof;
(ii) decreasing IgE levels in a subject in need thereof; or
(iii) preventing or treating allergic disorders in a subject in need thereof,
in which a therapeutically effective amount of said pharmaceutical composition is administered to the subject.

## Patentansprüche

1. Therapeutisch wirksame Menge eines Anti-Dectin-1-Antikörpers oder eines Antigen-bindenden Fragments davon, der/das funktionell mit Pam3CSK4 verbunden ist, zur Verwendung in einem Verfahren zum Verhindern oder Behandeln von allergischen oder entzündlichen Erkrankungen in einem Subjekt, das dessen bedarf, umfassend das Verabreichen an das Subjekt der therapeutisch wirksamen Menge des Anti-Dectin-1-Antikörpers oder des Antigen-bindenden Fragments davon, der/das funktionell mit Pam3CSK4 verbunden ist.

2. Anti-Dectin-1-Antikörper oder Antigen-bindendes Fragment davon, der/das funktionell mit Pam3CSK4 verbunden ist, zur Verwendung nach Anspruch 1, wobei:
(i) Pam3CSK4 an den Anti-Dectin-1-Antikörper oder das Antigen-bindende Fragment davon konjugiert ist; und/oder
(ii) Pam3CSK4 chemisch an den Anti-Dectin-1-Antikörper oder das Antigen-bindende Fragment davon konjugiert ist; und/oder
(iii) der Antikörper oder das Antigen-bindende Fragment spezifisch an Dectin-1 bindet und Dectin-1 aktiviert; und/oder
(iv) der Anti-Dectin-1-Antikörper oder das Antigen-bindende Fragment davon ein humaner Antikörper, humanisierter Antikörper, rekombinanter Antikörper, chimärer Antikörper, ein Antikörperderivat, ein furnierter Antikörper, ein Diabody, ein monoklonaler Antikörper oder ein polyklonaler Antikörper ist.

3. Anti-Dectin-1-Antikörper oder Antigen-bindendes Fragment davon, der/das funktionell mit Pam3CSK4 verbunden ist, zur Verwendung nach Anspruch 1 oder Anspruch 2, wobei:
(i) der Antikörper eine variable Region umfasst, die eine Aminosäuresequenz umfasst, ausgewählt aus den Sequenzen von SEQ ID NO: 2, 4, 6, 8, 10 und 12; oder
(ii) der Antikörper eine CDR umfasst, die eine Aminosäuresequenz hat, die einer beliebigen aus SEQ ID NO: 13-30 entspricht; oder
(iii) der Antikörper eine schwere Kette, die CDR umfasst, die eine Aminosäuresequenz hat, die SEQ ID NO: 13-15 entspricht, und eine leichte Kette umfasst, die eine Aminosäuresequenz hat, die SEQ ID NO: 16-18 entspricht; oder
(iv) der Antikörper eine schwere Kette, die CDR umfasst, die eine Aminosäuresequenz hat, die SEQ ID NO: 19-21 entspricht, und eine leichte Kette umfasst, die eine Aminosäuresequenz hat, die SEQ ID NO: 22-24 entspricht; oder
(v) der Antikörper eine schwere Kette, die CDR umfasst, die eine Aminosäuresequenz hat, die SEQ ID NO: 25-27 entspricht, und eine leichte Kette umfasst, die eine Aminosäuresequenz hat, die SEQ ID NO: 28-30 entspricht; oder
(vi) der Antikörper eine schwere oder leichte Kette mit einer Aminosäuresequenz umfasst, ausgewählt aus den Sequenzen von SEQ ID NO: 1, 3, 5, 7, 9 und 11; oder
(vii) der Antikörper oder das Antigen-bindende Fragment ferner eine Modifikation umfasst und die Modifikation Folgendes ist:
eine konservative Aminosäuremutation in den VH- und/oder VL-CDR1-, -CDR2- und/oder -CDR3-Regionen; oder
eine konservative Aminosäuremutation in der Fc-Scharnierregion; oder
Pegylierung; oder
Konjugation an ein Serumprotein; oder
Konjugation an Humanserumalbumin; oder
Konjugation an eine nachweisbare Markierung; oder
Konjugation an ein Diagnostikum; oder
Konjugation an ein Enzym; oder
Konjugation an ein fluoreszierendes, lumineszierendes oder biolumineszierendes Material; oder
Konjugation an ein radioaktives Material; oder
Konjugation an ein Therapeutikum.

4. Anti-Dectin-1-Antikörper oder Antigen-bindendes Fragment davon, der/das funktionell mit Pam3CSK4 verbunden ist, zur Verwendung nach einem vorhergehenden Anspruch, wobei:
(i) der Anti-Dectin-1-Antikörper oder das Antigen-bindende Fragment davon eine konstante γ4-Region umfasst; und/oder
(ii) der Anti-Dectin-1-Antikörper oder das Antigen-bindende Fragment davon eine konstante γ4-Region umfasst und die konstante γ4-Region eine Substitution von Glutaminsäure für Leucin an Rest 235 umfasst; und/oder
(iii) der Anti-Dectin-1-Antikörper oder das Antigen-bindende Fragment davon eine konstante γ4-Region umfasst und die konstante γ4-Region eine Substitution von Prolin für Serin an Rest 228 in der Scharnierregion umfasst; und/oder
(iv) das Subjekt ein Mensch ist; und/oder
(v) die Behandlung für eine allergische Erkrankung ist; und/oder
(vi) die Behandlung für eine TH-2-vermittelte allergische Erkrankung ist; und/oder
(vii) die Behandlung für eine TH-2-vermittelte entzündliche Erkrankung ist; und/oder
(viii) die Behandlung für eine TH-2-vermittelte entzündliche Erkrankung ist, die ausgewählt ist z. B. aus Asthma, chronisch obstruktiver Ventilationsstörung, interstitieller Lungenerkrankung, chronisch obstruktiver Lungenerkrankung, chronischer Bronchitis, eosinophiler Bronchitis, eosinophiler Pneumonie, Pneumonie, entzündlicher Darmerkrankung, atopischer Dermatitis, Atopie, Allergie, allergischer Rhinitis, idiopathischer Lungenfibrose, Sklerodermie, Emphysem, Brustkrebs und Colitis ulcerosa; und/oder
(ix) die Behandlung für eine TH-2-vermittelte entzündliche Erkrankung ist, die Brustkrebs ist; und/oder
(x) die Behandlung für eine TH-2-vermittelte entzündliche Erkrankung ist, die Colitis ulcerosa ist; und/oder
(xi) das Subjekt an Diabetes Typ 2 leidet oder unter dem Risiko steht, daran zu leiden; und/oder
(xii) das Subjekt eine allergische Erkrankung hat und der Antikörper oder das Antigen-bindende Fragment davon, der/das funktionell mit Pam3CSK4 verbunden ist, vor dem Ausbruch einer allergischen Reaktion verabreicht wird; oder das Subjekt eine allergische Erkrankung hat und der Antikörper oder das Antigen-bindende Fragment davon nach dem Ausbruch einer allergischen Reaktion verabreicht wird; und/oder
(xiii) der Anti-Dectin-1-Antikörper oder das Antigen-bindende Fragment davon, der/das funktionell mit Pam3CSK4 verbunden ist, in einer Menge verabreicht wird, die für die Zunahme von einem oder mehreren aus Th1, Th17 und Treg-Zellen in dem Subjekt wirksam ist; und/oder
(xiv) der Anti-Dectin-1-Antikörper oder das Antigen-bindende Fragment davon, der/das funktionell mit Pam3CSK4 verbunden ist, durch eine intradermale Injektion oder durch eine intravenöse Injektion verabreicht wird; und/oder
(xv) der Antikörper oder das Antigen-bindende Fragment davon ferner eine Modifikation umfasst; und/oder
(xvi) der Anti-Dectin-1-Antikörper oder das Antigen-bindende Fragment davon, der/das funktionell mit Pam3CSK4 verbunden ist, in einer pharmazeutischen Zusammensetzung verabreicht wird; und/oder
(xvii) der Anti-Dectin-1-Antikörper oder das Antigen-bindende Fragment davon, der/das funktionell mit Pam3CSK4 verbunden ist, in einer pharmazeutischen Zusammensetzung verabreicht wird und die pharmazeutische Zusammensetzung kein Antigen oder Allergen enthält; und/oder
(xviii) der Antikörper in einer pharmazeutischen Zusammensetzung verabreicht wird und die pharmazeutische Zusammensetzung im Wesentlichen aus einem Anti-Dectin-1-Antikörper oder einem Antigen-bindenden Fragment davon besteht, der/das funktionell mit Pam3CSK4 verbunden ist; und/oder
(xix) der Antikörper oder das Antigen-bindende Fragment davon nicht an ein Antigen konjugiert ist; und/oder
(xx) der Antikörper nicht an ein Dockerin- oder Cohesin-Molekül konjugiert ist.

5. Pharmazeutische Zusammensetzung, umfassend eine therapeutisch wirksame Menge eines Anti-Dectin-1-Antikörpers oder eines Antigen-bindenden Fragments davon, der/das funktionell mit Pam3CSK4 verbunden ist.

6. Pharmazeutische Zusammensetzung nach Anspruch 5, wobei:
(i) Pam3CSK4 an den Anti-Dectin-1-Antikörper oder das Antigen-bindende Fragment davon konjugiert ist; und/oder
(ii) der Antikörper oder das Antigen-bindende Fragment spezifisch an Dectin-1 bindet und Dectin-1 aktiviert; und/oder
(iii) der Antikörper oder das Antigen-bindende Fragment davon an humanes Dectin-1 bindet; und/oder
(iv) der Anti-Dectin-1-Antikörper oder das Antigen-bindende Fragment davon ein humaner Antikörper, humanisierter Antikörper, rekombinanter Antikörper, chimärer Antikörper, ein Antikörperderivat, ein furnierter Antikörper, ein Diabody, ein monoklonaler Antikörper oder ein polyklonaler Antikörper ist; und/oder
(v) der Anti-Dectin-1-Antikörper oder das Antigen-bindende Fragment davon eine konstante γ4-Region umfasst; und/oder
(vi) der Anti-Dectin-1-Antikörper oder das Antigen-bindende Fragment davon eine konstante γ4-Region umfasst und die konstante γ4-Region eine Substitution von Glutaminsäure für Leucin an Rest 235 umfasst; und/oder
(vii) der Anti-Dectin-1-Antikörper oder das Antigen-bindende Fragment davon eine konstante γ4-Region umfasst und die konstante γ4-Region eine Substitution von Prolin für Serin an Rest 228 in der Scharnierregion umfasst; und/oder
(viii) der Anti-Dectin-1-Antikörper oder das Antigen-bindende Fragment davon, der/das funktionell mit Pam3CSK4 verbunden ist, in einer Menge verabreicht wird, die für die Zunahme von einem oder mehreren aus Th1, Th17 und Treg-Zellen in dem Subjekt wirksam ist; und/oder
(ix) die Zusammensetzung zur intradermalen Injektion formuliert ist; und/oder
(x) die Zusammensetzung zur intravenösen Injektion formuliert ist; und/oder
(xi) der Antikörper oder das Antigen-bindende Fragment davon ferner eine Modifikation umfasst; und/oder
(xii) die pharmazeutische Zusammensetzung kein Antigen oder Allergen enthält; und/oder
(xiii) die pharmazeutische Zusammensetzung im Wesentlichen aus einem Anti-Dectin-1-Antikörper oder einem Antigen-bindenden Fragment davon besteht, der/das funktionell mit Pam3CSK4 verbunden ist; und/oder
(xiv) der Antikörper oder das Antigen-bindende Fragment davon nicht an ein Antigen konjugiert ist; und/oder
(xv) der Antikörper nicht an ein Dockerin- oder Cohesin-Molekül konjugiert ist.

7. Pharmazeutische Zusammensetzung nach Anspruch 6(i), wobei Pam3CSK4 chemisch an den Anti-Dectin-1-Antikörper oder das Antigen-bindende Fragment davon konjugiert ist.

8. Pharmazeutische Zusammensetzung nach einem der Ansprüche 5-7, wobei der Antikörper eine variable Region umfasst, die eine Aminosäuresequenz umfasst, ausgewählt aus den Sequenzen von SEQ ID NO: 2, 4, 6, 8, 10 und 12.

9. Pharmazeutische Zusammensetzung nach einem der Ansprüche 5-7, wobei der Antikörper eine CDR umfasst, die eine Aminosäuresequenz hat, die einer beliebigen aus SEQ ID NO: 13-30 entspricht.

10. Pharmazeutische Zusammensetzung nach einem der Ansprüche 5-9, wobei:
(i) der Antikörper eine schwere Kette, die CDR umfasst, die eine Aminosäuresequenz hat, die SEQ ID NO: 13-15 entspricht, und eine leichte Kette umfasst, die eine Aminosäuresequenz hat, die SEQ ID NO: 16-18 entspricht; oder
(ii) der Antikörper eine schwere Kette, die CDR umfasst, die eine Aminosäuresequenz hat, die SEQ ID NO: 19-21 entspricht, und eine leichte Kette umfasst, die eine Aminosäuresequenz hat, die SEQ ID NO: 22-24 entspricht; oder
(iii) der Antikörper eine schwere Kette, die CDR umfasst, die eine Aminosäuresequenz hat, die SEQ ID NO: 25-27 entspricht, und eine leichte Kette umfasst, die eine Aminosäuresequenz hat, die SEQ ID NO: 28-30 entspricht.

11. Pharmazeutische Zusammensetzung nach einem der Ansprüche 5-9, wobei der Antikörper eine schwere oder leichte Kette mit einer Aminosäuresequenz umfasst, ausgewählt aus den Sequenzen von SEQ ID NO: 1, 3, 5, 7, 9 und 11.

12. Pharmazeutische Zusammensetzung nach Anspruch 6(xi), wobei die Modifikation Folgendes ist:
eine konservative Aminosäuremutation in den VH- und/oder VL-CDR1-, -CDR2- und/oder -CDR3-Regionen; oder
eine konservative Aminosäuremutation in der Fc-Scharnierregion; oder
Pegylierung; oder
Konjugation an ein Serumprotein; oder
Konjugation an Humanserumalbumin; oder
Konjugation an eine nachweisbare Markierung; oder
Konjugation an ein Diagnostikum; oder
Konjugation an ein Enzym; oder
Konjugation an ein fluoreszierendes, lumineszierendes oder biolumineszierendes Material; oder
Konjugation an ein radioaktives Material; oder
Konjugation an ein Therapeutikum.

13. Pharmazeutische Zusammensetzung nach einem der Ansprüche 6-12 zur Verwendung in einem Verfahren zum:
(i) Verringern von Th2-Typ-Zellantworten in einem Subjekt, das dessen bedarf;
(ii) Verringern von IgE-Werten in einem Subjekt, das dessen bedarf; oder
(iii) Verhindern oder Behandeln von allergischen Erkrankungen in einem Subjekt, das dessen bedarf, wobei eine therapeutisch wirksame Menge der pharmazeutischen Zusammensetzung an das Subjekt verabreicht wird.

## Revendications

1. Quantité thérapeutiquement efficace d'un anticorps anti-dectine-1 ou fragment de liaison à un antigène de celui-ci fonctionnellement liée à Pam3CSK4, pour une utilisation dans un procédé visant à prévenir ou traiter des troubles allergiques et inflammatoires chez un sujet qui le nécessite, comprenant l'administration au sujet de la quantité thérapeutiquement efficace de l'anticorps anti-dectine-1 ou fragment de liaison à un antigène de celui-ci fonctionnellement lié à Pam3CSK4.

2. Anticorps anti-dectine-1 ou fragment de liaison à un antigène de celui-ci fonctionnellement lié à Pam3CSK4, pour une utilisation selon la revendication 1, dans laquelle :
(i) Pam3CSK4 est conjugué à l'anticorps anti-dectine-1 ou fragment de liaison à un antigène de celui-ci ; et/ou
(ii) Pam3CSK4 est chimiquement conjugué à l'anticorps anti-dectine-1 ou fragment de liaison à un antigène de celui-ci ; et/ou
(iii) l'anticorps anti-dectine-1 ou fragment de liaison à un antigène de celui-ci se lie spécifiquement à la dectine-1 et active la dectine-1 ; et/ou
(iv) l'anticorps anti-dectine-1 ou fragment de liaison à un antigène de celui-ci est un anticorps humain, un anticorps humanisé, un anticorps recombinant, un anticorps chimérique, un dérivé d'anticorps, un anticorps plaqué, un diabody, un anticorps monoclonal ou un anticorps polyclonal.

3. Anticorps anti-dectine-1 ou fragment de liaison à un antigène de celui-ci fonctionnellement lié à Pam3CSK4, pour une utilisation selon la revendication 1 ou la revendication 2, dans laquelle :
(i) l'anticorps comprend une région variable comprenant une séquence d'acides aminés choisie parmi les séquences de SEQ ID N° : 2, 4, 6, 8, 10 et 12 ; ou
(ii) l'anticorps comprend une CDR ayant une séquence d'acides aminés correspondant à l'une quelconque des SEQ N° : 13 à 30 ; ou
(iii) l'anticorps comprend une chaîne lourde ayant des CDR ayant une séquence d'acides aminés correspondant à SEQ ID N° : 13 à 15 et une chaîne légère ayant une séquence d'acides aminés correspondant à SEQ ID N° : 16 à 18 ; ou
(iv) l'anticorps comprend une chaîne lourde ayant des CDR ayant une séquence d'acides aminés correspondant à SEQ ID N° : 19 à 21 et une chaîne légère ayant une séquence d'acides aminés correspondant à SEQ ID N° : 22 à 24 ; ou
(v) l'anticorps comprend une chaîne lourde ayant des CDR ayant une séquence d'acides aminés correspondant à SEQ ID N° : 25 à 27 et une chaîne légère ayant une séquence d'acides aminés correspondant à SEQ ID N° : 28 à 30 ; ou
(vi) l'anticorps comprend une chaîne lourde ou légère avec une séquence d'acides aminés choisie parmi les séquences de SEQ ID N° : 1, 3, 5, 7, 9 et 11 ; ou
(vii) l'anticorps ou fragment de liaison à un antigène comprend en outre une modification et la modification est :
une mutation d'acide aminé conservatrice dans les régions CDR 1, CDR 2 et/ou CDR 3 de VH et/ou de VL ; ou
une mutation d'acide aminé conservatrice dans la région charnière Fc ; ou
une pégylation ; ou
une conjugaison à une protéine sérique ; ou
une conjugaison à l'albumine sérique humaine ; ou
une conjugaison à un marqueur détectable ; ou
une conjugaison à un agent de diagnostic ; ou
une conjugaison à une enzyme ; ou
une conjugaison à une matière fluorescente, luminescente ou bioluminescente ; ou
une conjugaison à une matière radioactive ; ou
une conjugaison à un agent thérapeutique.

4. Anticorps anti-dectine-1 ou fragment de liaison à un antigène de celui-ci fonctionnellement lié à Pam3CSK4, pour une utilisation selon l'une quelconque des revendications précédentes, dans laquelle :
(i) l'anticorps anti-dectine-1 ou fragment de liaison à un antigène de celui-ci comprend une région constante γ4 ; et/ou
(ii) l'anticorps anti-dectine-1 ou fragment de liaison à un antigène de celui-ci comprend une région constante γ4 et la région constante γ4 comprend une substitution d'acide glutamique par la leucine au niveau du résidu 235 ; et/ou
(iii) l'anticorps anti-dectine-1 ou fragment de liaison à un antigène de celui-ci comprend une région constante γ4 et la région constante γ4 comprend une substitution de proline par la sérine au niveau du résidu 228 dans la région charnière ; et/ou
(iv) le sujet est un sujet humain : et/ou
(v) le traitement vise un trouble allergique ; et/ou
(vi) le traitement vise un trouble allergique médié par TH2 ; et/ou
(vii) le traitement vise un trouble inflammatoire médié par TH2 ; et/ou
(viii) le traitement vise un trouble inflammatoire médié par TH2 qui est choisi parmi l'asthme, la bronchopneumopathie chronique obstructive, la pneumopathie interstitielle, la bronchopneumopathie chronique obstructive, la bronchite chronique, la bronchite éosinophile, la pneumonie éosinophile, la pneumonie, une maladie inflammatoire de l'intestin, la dermatite atopique, l'atopie, l'allergie, la rhinite allergique, la fibrose pulmonaire idiopathique, la sclérodermie, l'emphysème, le cancer du sein et la rectocolite hémorragique ; et/ou
(ix) le traitement vise un trouble inflammatoire médié par TH2 qui est le cancer du sein ; et/ou
(x) le traitement vise un trouble inflammatoire médié par TH2 qui est la rectocolite hémorragique ; et/ou
(xi) le sujet est atteint ou présente un risque d'être atteint de diabète de type 2 ; et/ou
(xii) le sujet présente un trouble allergique et l'anticorps ou fragment de liaison à un antigène de celui-ci fonctionnellement lié à Pam3CSK4 est administré avant le déclenchement d'une réaction allergique ; ou le sujet présente un trouble allergique et l'anticorps ou fragment de liaison à un antigène de celui-ci est administré après le déclenchement d'une réaction allergique ; et/ou
(xiii) l'anticorps anti-dectine-1 ou fragment de liaison à un antigène de celui-ci fonctionnellement lié à Pam3CSK4 est administré dans une quantité efficace pour l'augmentation d'au moins les unes de cellules Th1, Th17 et Treg chez le sujet ; et/ou
(xiv) l'anticorps anti-dectine-1 ou fragment de liaison à un antigène de celui-ci fonctionnellement lié à Pam3CSK4 est administré par injection intradermique ou par injection intraveineuse ; et/ou
(xv) l'anticorps ou fragment de liaison à un antigène comprend en outre une modification ; et/ou
(xvi) l'anticorps anti-dectine-1 ou fragment de liaison à un antigène de celui-ci fonctionnellement lié à Pam3CSK4 est administré dans une composition pharmaceutique ; et/ou
(xvii) l'anticorps anti-dectine-1 ou fragment de liaison à un antigène de celui-ci fonctionnellement lié à Pam3CSK4 est administré dans une composition pharmaceutique et la composition pharmaceutique ne contient pas d'antigène ou d'allergène ; et/ou
(xviii) l'anticorps est administré dans une composition pharmaceutique et la composition pharmaceutique est essentiellement constituée d'un anticorps anti-dectine-1 ou fragment de liaison à un antigène de celui-ci fonctionnellement lié à Pam3CSK4 ; et/ou
(xix) l'anticorps ou fragment de liaison à un antigène de celui-ci n'est pas conjugué à un antigène ; et/ou
(xx) l'anticorps n'est pas conjugué à une molécule de dockérine ou de cohésine.

5. Composition pharmaceutique comprenant une quantité thérapeutiquement efficace d'un anticorps anti-dectine-1 ou fragment de liaison à un antigène de celui-ci fonctionnellement lié à Pam3CSK4.

6. Composition pharmaceutique de la revendication 5, dans laquelle :
(i) Pam3CSK4 est conjugué à l'anticorps anti-dectine-1 ou fragment de liaison à un antigène de celui-ci ; et/ou
(ii) l'anticorps ou fragment de liaison à un antigène se lie spécifiquement à la dectine-1 et active la dectine-1 ; et/ou
(iii) l'anticorps ou fragment de liaison à un antigène se lie à la dectine-1 humaine ; et/ou
(iv) l'anticorps anti-dectine-1 ou fragment de liaison à un antigène de celui-ci est un anticorps humain, un anticorps humanisé, un anticorps recombinant, un anticorps chimérique, un dérivé d'anticorps, un anticorps plaqué, un diabody, un anticorps monoclonal ou un anticorps polyclonal ; et/ou
(v) l'anticorps anti-dectine-1 ou fragment de liaison à un antigène de celui-ci comprend une région constante γ4 ; et/ou
(vi) l'anticorps anti-dectine-1 ou fragment de liaison à un antigène de celui-ci comprend une région constante γ4 et la région constante γ4 comprend une substitution d'acide glutamique par la leucine au niveau du résidu 235 ; et/ou
(vii) l'anticorps anti-dectine-1 ou fragment de liaison à un antigène de celui-ci comprend une région constante γ4 et la région constante γ4 comprend une substitution de proline par la sérine au niveau du résidu 228 dans la région charnière ; et/ou
(viii) l'anticorps anti-dectine-1 ou fragment de liaison à un antigène de celui-ci fonctionnellement lié à Pam3CSK4 est dans une quantité efficace pour l'augmentation d'au moins les unes de cellules Th1, Th17 et Treg chez le sujet ; et/ou
(ix) la composition est formulée pour une injection intradermique ; et/ou
(x) la composition est formulée pour une injection intraveineuse ; et/ou
(xi) l'anticorps ou fragment de liaison à un antigène comprend en outre une modification ; et/ou
(xii) la composition pharmaceutique ne contient pas d'antigène ou d'allergène ; et/ou
(xiii) la composition pharmaceutique est essentiellement constituée d'un anticorps anti-dectine-1 ou fragment de liaison à un antigène de celui-ci fonctionnellement lié à Pam3CSK4 ; et/ou
(xiv) l'anticorps ou fragment de liaison à un antigène de celui-ci n'est pas conjugué à un antigène ; et/ou
(xv) l'anticorps n'est pas conjugué à une molécule de dockérine ou de cohésine.

7. Composition pharmaceutique de la revendication 6(i), dans laquelle Pam3CSK4 est chimiquement conjugué à l'anticorps anti-dectine-1 ou fragment de liaison à un antigène de celui-ci.

8. Composition pharmaceutique de l'une quelconque des revendications 5 à 7, dans laquelle l'anticorps comprend une région variable comprenant une séquence d'acides aminés choisie parmi les séquences de SEQ ID N° : 2, 4, 6, 8, 10 et 12.

9. Composition pharmaceutique de l'une quelconque des revendications 5 à 7, dans laquelle l'anticorps comprend une CDR ayant une séquence d'acides aminés correspondant à l'une quelconque des SEQ N° : 13 à 30.

10. Composition pharmaceutique de l'une quelconque des revendications 5 à 9, dans laquelle :
(i) l'anticorps comprend une chaîne lourde ayant des CDR ayant une séquence d'acides aminés correspondant à SEQ ID N° : 13 à 15 et une chaîne légère ayant une séquence d'acides aminés correspondant à SEQ ID N° : 16 à 18 ; ou
(ii) l'anticorps comprend une chaîne lourde ayant des CDR ayant une séquence d'acides aminés correspondant à SEQ ID N° : 19 à 21 et une chaîne légère ayant une séquence d'acides aminés correspondant à SEQ ID N° : 22 à 24 ; ou
(iii) l'anticorps comprend une chaîne lourde ayant des CDR ayant une séquence d'acides aminés correspondant à SEQ ID N° : 25 à 27 et une chaîne légère ayant une séquence d'acides aminés correspondant à SEQ ID N° : 28 à 30.

11. Composition pharmaceutique de l'une quelconque des revendications 5 à 9, dans laquelle l'anticorps comprend une chaîne lourde ou légère avec une séquence d'acides aminés issue des séquences de SEQ ID N° : 1, 3, 5, 7, 9 et 11.

12. Composition pharmaceutique de la revendication 6(xi), dans laquelle la modification est :
une mutation d'acide aminé conservatrice dans les régions CDR 1, CDR 2 et/ou CDR 3 de VH et/ou de VL ; ou
une mutation d'acide aminé conservatrice dans la région charnière Fc ; ou
une pégylation ; ou
une conjugaison à une protéine sérique ; ou
une conjugaison à l'albumine sérique humaine ; ou
une conjugaison à un marqueur détectable ; ou
une conjugaison à un agent de diagnostic ; ou
une conjugaison à une enzyme ; ou
une conjugaison à une matière fluorescente, luminescente ou bioluminescente ; ou
une conjugaison à une matière radioactive ; ou
une conjugaison à un agent thérapeutique.

13. Composition pharmaceutique selon l'une quelconque des revendications 6 à 12, pour à une utilisation dans un procédé visant à :
(i) réduire les réponses de cellules Th2 chez un sujet qui le nécessite ;
(ii) réduire le taux d'IgE chez un sujet qui le nécessite ; ou
(iii) prévenir ou traiter des troubles allergiques chez un sujet qui le nécessite,
dans lequel une quantité thérapeutiquement efficace de ladite composition pharmaceutique est administrée au sujet.
